# EUROPEAN PATENT SPECIFICATION

(11) **EP 4 021 356 B1**
(45) Date of publication and mention of the grant of the patent: **12.02.2025**
(21) Application number: 20776260.0
(22) Date of filing: 28.08.2020
(51) Int. Cl.: A61F 5/56

(54) **SYSTEMS AND METHODS FOR ADJUSTING USER POSITION USING MULTI-COMPARTMENT BLADDERS**
SYSTEME UND VERFAHREN ZUR EINSTELLUNG EINER BENUTZERPOSITION UNTER VERWENDUNG VON MEHRKAMMERBLASEN
SYSTÈMES ET PROCÉDÉS DE RÉGLAGE DE LA POSITION D'UN UTILISATEUR À L'AIDE DE VESSIES À PLUSIEURS COMPARTIMENTS

(30) Priority: 30.08.2019 US 201962894404 P
(43) Date of publication of application: 06.07.2022
(73) Proprietor: Resmed Corp., San Diego, CA 92123 (US); ResMed Sensor Technologies Limited, Sandyford D18 T6T7 Dublin (IE); ResMed Pty Ltd, Bella Vista, New South Wales 2153 (AU)
(72) Inventor: PINCZUK, Michael, San Diego, California 92123 (US); GRENNAN, Kieran, Dublin D18 T6T7 (IE); LAW, Ian, Bella Vista, New South Wales 2153 (AU)
(74) Representative: Mathys & Squire
(86) International application number: PCT/US2020/048633
(87) International publication number: WO 2021/041987

(56) References cited:
- EP-A2- 1 844 743
- WO-A1-2016/054949
- KR-A- 20120 056 655

## Description

### TECHNICAL FIELD

The present disclosure relates generally to systems and methods for treating respiratory events during a sleep session, and more particularly, to systems and methods for adjusting a user position during a sleep session using a multi-compartment bladder.

### BACKGROUND

Many individuals suffer from sleep-related respiratory disorders that cause one or more events during a sleep session, such as, for example, snoring, an apnea, a hypopnea, a restless leg, a sleeping disorder, choking, an increased heart rate, labored breathing, an asthma attack, an epileptic episode, a seizure, or any combination etc. While the occurrence of these events can be eliminated or substantially reduced using a continuous positive airway pressure (CPAP) system, all or some of these events can be eliminated or reduced by adjusting a position of the individual during a sleep session. EP 1.844.743 (which is the closest prior art document) discloses an apparatus for preventing a sleeping respiratory obstruction, comprising: a pillow sheet having a plurality of chambers on which a body of a user is supported; and a control module for controlling inflation and deflation of the chambers supporting the body of the user in order to open an upper airway of the user being subjected to the sleep respiratory obstruction during sleep.

The present disclosure is directed to solving these and other problems.

### SUMMARY

According to some implementations of the present disclosure, a system includes one or more sensors, a multi-compartment bladder, a memory, and a control system. The one or more sensors are configured to generate data associated with a sleep session of a user. The multi-compartment bladder us configured to be positioned adjacent to the user during the sleep session. The memory stores machine-readable instructions. The control system includes one or more processors configured to execute the machine-readable instructions to determine, based at least in part on the generated data associated with the sleep session, that the user is experiencing or has experienced an event and responsive to determining that the user is experiencing or has experienced the event, cause the multi-compartment bladder to be modified to aid in causing the user to move.

According to some implementations of the present disclosure, a method includes receiving, from one or more sensors, data associated with a sleep session of a user. The method also includes determining, based at least in part on the data, that the user is experiencing or has experienced an event during the sleep session. The method also includes causing a multi-compartment bladder that is positioned adjacent to the user to be modified aid in causing the user to move responsive to determining that the user is experiencing or has experienced the event.

According to some implementations of the present disclosure, a system includes a sensor, a multi-compartment bladder, a memory, and a control system. The sensor is configured to generate data associated with a sleep session of a user. The multi-compartment bladder is configured to be positioned adjacent to a user during the sleep session. The memory stores machine-readable instructions. The control system including one or more processors configured to execute the machine-readable instructions to analyze the generated data associated with the sleep session of the user; determine, based at least in part on the analysis, that the user is experiencing or has experienced an event and responsive to the determination that the user is experiencing or has experienced the event, cause the multi-compartment bladder to be modified according to first inflation scheme to aid in causing the user to move.

According to some implementations of the present disclosure, a system for aiding in adjusting a user position during a sleep session includes a respiratory device, a mask, a pump, a sensor, a memory, and a control system. The mask is configured to be coupled to the user during the sleep session to aid in supplying pressurized air to the user. The multi-compartment bladder is configured to be positioned adjacent to a pillow used by the user during the sleep session. The multi-compartment bladder has at least two separate and distinct compartments. The pump device is configured to at least partially inflate and at least partially deflate each of the at least two separate and distinct compartments. The sensor is configured to determine a location of a head of the user on the pillow during the sleep session. The memory stores machine-readable instructions, a control system including one or more processors configured to execute the machine-readable instructions to: responsive to determining that an event has occurred determine a location of the head of the user on the pillow using the at least one sensor. The control system is further configured to, based at least in part on the determined location of the head, using the pump, at least partially inflate a first one of the at least two separate and distinct compartments and at least partially deflate a second one of the at least two separate and distinct compartments to aid in causing the head of the user to move.

According to some implementations of the present disclosure, a system includes a respirator device, an interface, a sensor, a pad, a memory, and a control system. The respirator device is configured to supply pressurized air. The interface is coupled to the respirator device via a conduit and is being configured to engage a user during a sleep session to aid in directing the supplied pressurized air to an airway of the user. The sensor is configured to generate physiological data associated with the user. The pad is configured to be positioned adjacent to the user during the sleep session. The memory stores machine-readable instructions. The control system including one or more processors configured to execute the machine-readable instructions to analyze the physiological data to determine a sleep state of the user and based on the determined sleep state of the user, modify a temperature setting of the pad.

According to some implementations of present disclosure, a system includes a sensor, a multi-compartment bladder, a memory, and a control system. The sensor is configured to generate data associated with a sleep session of a user. The multi-compartment bladder is configured to be positioned adjacent to a user during the sleep session. The memory stores machine-readable instructions. The control system includes one or more processors configured to execute the machine-readable instructions to receive an input indicative of an initial fill scheme for the multi-compartment bladder. The control system is further configured to, responsive to the received input, cause the multi-compartment bladder to be filled according to the initial fill scheme, analyze the generated data associated with the sleep session of the user, and responsive to the analysis, cause the multi-compartment bladder to be filled according to a second fill scheme.

According to some implementations of the present disclosure, a system includes a respirator device configured to (i) supply pressurized air and (ii) generate data associated with a sleep session of a user, a mask coupled to the respirator device via a tube and being configured to engage the user during the sleep session to aid in directing the supplied pressurized air to an airway of the user, a multi-compartment bladder configured to be positioned adjacent to a user during the sleep session, a memory storing machine-readable instructions, and a control system including one or more processors configured to execute the machine-readable instructions to: analyze the generated data associated with the sleep session of the user, determine, based at least in part on the analysis, that the user is experiencing or has experienced an event, responsive to the determination that the user is experiencing or has experienced the event, cause the multi-compartment bladder to be modified to aid in causing the user to move, subsequent to the multi-compartment bladder being modified, continue to analyze the generated data to determine if the user is still experiencing the event, and responsive to the continued analysis of the generated data resulting in a determination that the user is still experiencing the event, cause a pressure setting of the respirator device to be increased.

According to some implementations of the present disclosure, a method includes receiving, from one or more sensors, physiological data associated with a user during a sleep session. The method also includes determining, based at least in part on the physiological data, a sleep state of the user during the sleep session. The method also includes modifying a temperature setting of a pad positioned adjacent to the user based at least in part on the determined sleep state of the user.

The above summary is not intended to represent each embodiment or every aspect of the present invention. Additional features and benefits of the present invention are apparent from the detailed description and figures set forth below.

### BRIEF DESCRIPTION OF THE DRAWINGS

FIG. 1 is a functional block diagram of a system for adjusting a user position during a sleep session, according to some implementations of the present disclosure;
FIG. 2 is a perspective view of at least a portion of the system of FIG. 1 and a first user, according to some implementations of the present disclosure;
FIG. 3 is a top view of a generally U-shaped multi-compartment bladder of the system of FIG. 1 with a plurality of compartments fluidly coupled in series, according to some implementations of the present disclosure;
FIG. 4 is a top view of a generally U-shaped multi-compartment bladder of the system of FIG. 1, according to some implementations of the present disclosure;
FIG. 5 is a top view of a generally rectangular multi-compartment bladder of the system of FIG. 1, according to some implementations of the present disclosure;
FIG. 6A is a partial cross-sectional view of the multi-compartment bladder of FIG. 3 and a side view of a user in a first position, according to some implementations of the present disclosure;
FIG. 6B is a partial cross-section view of the multi-compartment bladder of FIG. 3 and a side view of the user in a second position, according to some implementations of the present disclosure;
FIG. 6C is a partial cross-sectional view of the multi-compartment bladder of FIG. 3 and a side view of a user in a first position, according to some implementations of the present disclosure;
FIG. 6D is a partial cross-section view of the multi-compartment bladder of FIG. 3 and a side view of the user in a second position, according to some implementations of the present disclosure;
FIG. 7 is a process flow diagram for a method of aiding in adjusting a user position during a sleep session using a multi-compartment bladder, according to some implementations of the present disclosure;
FIG. 8 is a process flow diagram for a method of adjusting a multi-compartment bladder from an initial fill scheme to a second fill scheme, according to some implementations of the present disclosure;
FIG. 9 is a functional block diagram of a system for adjusting a temperature of a pad during a sleep session, according to some implementations of the present disclosure;
FIG. 10 is a process flow diagram for a method of adjusting a temperature of a pad based at least in part on data associated with a sleep session, according to some implementations of the present disclosure;
FIG. 11 illustrates an exemplary timeline for a sleep session, according to some implementations of the present disclosure; and
FIG. 12 illustrates an exemplary hypnogram associated with the sleep session of FIG. 11, according to some implementations of the present disclosure.

### DETAILED DESCRIPTION

Many individuals suffer from sleep-related respiratory disorders, such as Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), and chest wall disorders.

Obstructive Sleep Apnea (OSA) is a form of Sleep Disordered Breathing (SDB), and is characterized by events including occlusion or obstruction of the upper air passage during sleep resulting from a combination of an abnormally small upper airway and the normal loss of muscle tone in the region of the tongue, soft palate and posterior oropharyngeal wall. More generally, an apnea generally refers to the cessation of breathing caused by blockage of the air (Obstructive Sleep Apnea) or the stopping of the breathing function (often referred to as central apnea). Typically, the individual will stop breathing for between about 15 seconds and about 30 seconds during an obstructive sleep apnea event.

Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathing. Hypercapnia is generally characterized by elevated or excessive carbon dioxide in the bloodstream, typically caused by inadequate respiration.

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterized by repetitive deoxygenation and re-oxygenation of the arterial blood.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung.

Neuromuscular Disease (NMD) encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

These disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that occur when the individual is sleeping. Such events generally disrupt the quality of the individual's sleep, leading to the manifestation of physical symptoms and/or adverse health effects.

The Apnea-Hypopnea Index (AHI) is an index used to indicate the severity of sleep apnea during a sleep session. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

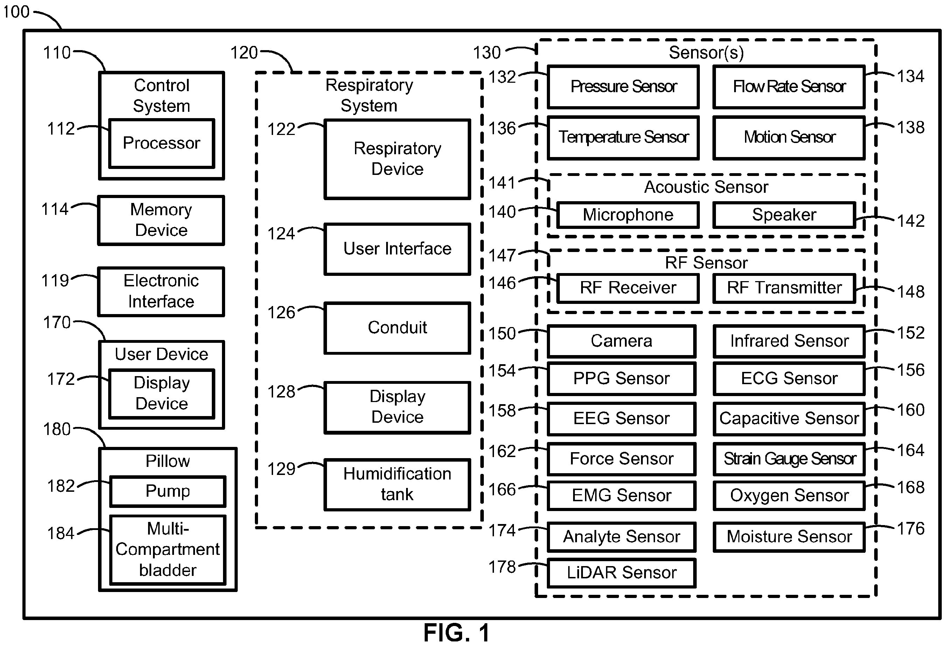

Referring to FIG. 1, a system 100 includes a control system 110, a memory device 114, a respiratory system 120, one or more sensors 130, a pillow 180, and a mobile device 170. As described herein, the system 100 generally can be used to adjust a position of a user during a sleep session.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 can be coupled to and/or positioned within, for example, a housing of the user device 170, a portion (e.g., a housing) of the respiratory system 120, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory device 122, within a housing of the user device 170, within a housing of one or more of the sensors 130, or any combination thereof. Like the control system 110, the memory device 114 can be centralized (within one such housing) or decentralized (within two or more of such housings, which are physically distinct).

Respiratory therapy systems (e.g., continuous positive airway pressure (CPAP) systems) are often used to treat individuals suffering from sleep-related respiratory disorders. Generally, the user of a respiratory therapy system wears an interface (e.g., mask) while sleeping, which delivers pressurized air into the user's throat to aid in preventing the airway from narrowing and/or collapsing and increasing the user's oxygen intake. However, forcing pressurized air into the user's throat can cause a variety of adverse effects, such as, for example, dryness in the nose and/or mouth, a sore throat, etc. The pressurized air can also force air into the stomach, causing general discomfort, bloating, and/or gas. As another example, the pressurized air can cause a vacuum effect leading to stomach reflux. If the interface between the user's face and the mask does not form a substantially air-tight seal, pressurized air can leak from the mask causing undesirable noise that may wake the user and/or their bed partner, dryness of the eyes and/or skin, and/or lower the volume of air being delivered to the user (e.g., which may necessitate an increase in pressure), thereby impeding the proper delivery of therapy. Air leakage from the mask can occur, for example, if the user is laying on their side as opposed to laying on their back while sleeping.

Some respiratory therapy systems attempt to mitigate these adverse side effects by humidifying and/or heating the pressurized air. However, it is generally desirable to deliver pressurized air to the individual only insofar as it is needed to prevent or substantially reduce the likelihood of an event, or to address an event that the user is currently experiencing. In many cases, merely changing the physical position of the user during sleep (e.g., moving the head up to open the airway) can prevent or reduce the occurrence of events and/or reduce the severity of the events, in particular in respect of positional obstructive sleep apnea wherein apneic episodes are associated with the user's sleep position and changing the physical position of the user can reduce or prevent such episodes. Changing the physical position of the user (e.g., rotating the user onto their back) can also aid in reducing or preventing air leakage from the mask. Thus, it would be desirable to automatically adjust a physical position of the user during a sleep session to reduce, eliminate, and/or address the occurrence of an event without the need for pressurized air from the respiratory therapy system, or as a further measure in addition to use of the respiratory therapy system. Further, even if adjusting the position of the user does not by itself reduce, eliminate, and/or address an event, the adjusted position can reduce the required pressure that needs to be delivered by the respiratory therapy system (e.g., 10% less pressure, 30% less pressure, 50% less pressure, 75% less pressure, etc.).

Referring to FIG. 1, the respiratory system 120 (also referred to as a respiratory therapy system) can include a respiratory pressure therapy device 122 (referred to herein as respiratory device 122), a user interface 124, a conduit 126 (also referred to as a tube or an air circuit), a display device 128, a humidification tank 129, or any combination thereof. In some implementations, the control system 110, the memory device 114, the display device 128, one or more of the sensors 130, and the humidification tank 129 are part of the respiratory device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory system 120 is generally used to treat individuals suffering from one or more sleep-related respiratory disorders (e.g., obstructive sleep apnea, central sleep apnea, or mixed sleep apnea).

The respiratory device 122 is generally used to generate pressurized air that is delivered to a user (e.g., using one or more motors that drive one or more compressors). In some implementations, the respiratory device 122 generates continuous constant air pressure that is delivered to the user. In other implementations, the respiratory device 122 generates two or more predetermined pressures (e.g., a first predetermined air pressure and a second predetermined air pressure). In still other implementations, the respiratory device 122 is configured to generate a variety of different air pressures within a predetermined range. For example, the respiratory device 122 can deliver at least about 6 cm H₂O, at least about 10 cm H₂O, at least about 20 cm H₂O, between about 6 cm H₂O and about 10 cm H₂O, between about 7 cm H₂O and about 12 cm H₂O, etc. The respiratory device 122 can also deliver pressurized air at a predetermined flow rate between, for example, about -20 L/min and about 150 L/min, while maintaining a positive pressure (relative to the ambient pressure).

The user interface 124 engages a portion of the user's face and delivers pressurized air from the respiratory device 122 to the user's airway to aid in preventing the airway from narrowing and/or collapsing during sleep. This may also increase the user's oxygen intake during sleep. Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 cm H₂O relative to ambient pressure. For other forms of therapy, such as the delivery of oxygen, the user interface may not include a seal sufficient to facilitate delivery to the airways of a supply of gas at a positive pressure of about 10 cm H₂O.

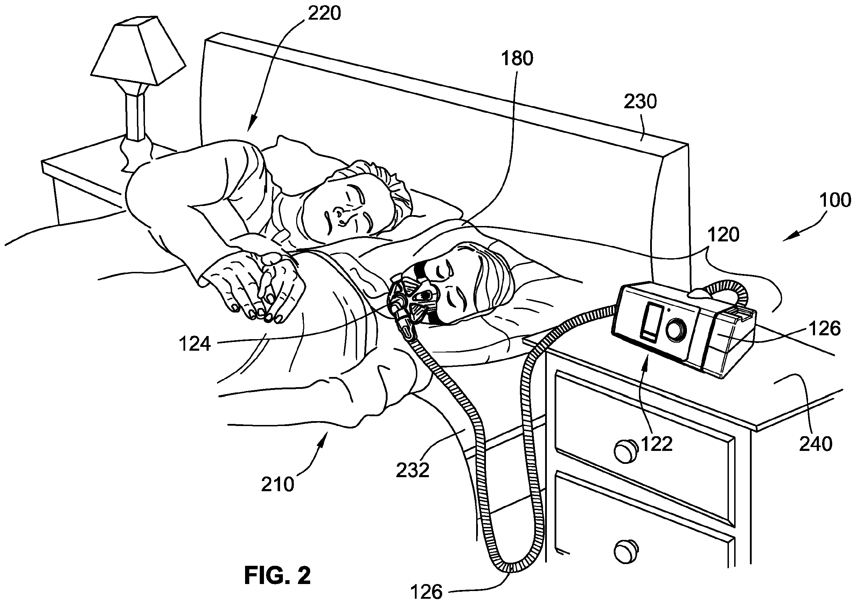

As shown in FIG. 2, in some implementations, the user interface 124 is a facial mask that covers the nose and mouth of the user. Alternatively, the user interface 124 can be a nasal mask that provides air to the nose of the user or a nasal pillow mask that delivers air directly to the nostrils of the user. The user interface 124 can include a plurality of straps (e.g., including hook and loop fasteners) for positioning and/or stabilizing the interface on a portion of the user (e.g., the face) and a conformal cushion (e.g., silicone, plastic, foam, etc.) that aids in providing an air-tight seal between the user interface 124 and the user. The user interface 124 can also include one or more vents for permitting the escape of carbon dioxide and other gases exhaled by the user 210. In other implementations, the user interface 124 can include a mouthpiece (e.g., a night guard mouthpiece molded to conform to the user's teeth, a mandibular repositioning device, etc.).

The conduit 126 (also referred to as an air circuit or tube) allows the flow of air between two components of a respiratory system 120, such as the respiratory device 122 and the user interface 124. In some implementations, there can be separate limbs of the conduit for inhalation and exhalation. In other implementations, a single limb conduit is used for both inhalation and exhalation.

One or more of the respiratory device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be use, for example, to measure the air pressure and/or flow rate of pressurized air supplied by the respiratory device 122.

The display device 128 is generally used to display image(s) including still images, video images, or both and/or information regarding the respiratory device 122. For example, the display device 128 can provide information regarding the status of the respiratory device 122 (e.g., whether the respiratory device 122 is on/off, the pressure of the air being delivered by the respiratory device 122, the temperature of the air being delivered by the respiratory device 122, etc.) and/or other information (e.g., a sleep score or a therapy score (e.g. myAir^{™} score)), the current date/time, personal information for the user 210, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory device 122.

The humidification tank 129 is coupled to or integrated in the respiratory device 122 and includes a reservoir of water that can be used to humidify the pressurized air delivered from the respiratory device 122. The respiratory device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user.

The respiratory system 120 can be used, for example, as a ventilator or a positive airway pressure (PAP) system such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Referring to FIG. 2, a portion of the system 100 (FIG. 1), according to some implementations, is illustrated. A user 210 of the respiratory system 120 and a bed partner 220 are located in a bed 230 and are laying on a mattress 232. The user interface 124 (e.g., a full facial mask) can be worn by the user 210 during a sleep session. The user interface 124 is fluidly coupled and/or connected to the respiratory device 122 via the conduit 126. In turn, the respiratory device 122 delivers pressurized air to the user 210 via the conduit 126 and the user interface 124 to increase the air pressure in the throat of the user 210 to aid in preventing the airway from closing and/or narrowing during sleep. The respiratory device 122 can be positioned on a nightstand 240 that is directly adjacent to the bed 230 as shown in FIG. 2, or more generally, on any surface or structure that is generally adjacent to the bed 230 and/or the user 210.

Referring to back to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, a RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a LiDAR sensor 178, or any combination thereof. Generally, each of the one or sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the infrared sensor 152, the photoplethysmogram (PPG) sensor 154, the electrocardiogram (ECG) sensor 156, the electroencephalography (EEG) sensor 158, the capacitive sensor 160, the force sensor 162, the strain gauge sensor 164, the electromyography (EMG) sensor 166, the oxygen sensor 168, the analyte sensor 174, the moisture sensor 176, and the LiDAR sensor 178, more generally, the one or more sensors 130 can include any combination and any number of each of the sensors described and/or shown herein.

The one or more sensors 130 can be used to generate, for example, physiological data, audio data, or both. Physiological data generated by one or more of the sensors 130 can be used by the control system 110 to determine a sleep-wake signal associated with a user during a sleep session and one or more sleep-related parameters. The sleep-wake signal can be indicative of one or more sleep states, including wakefulness, relaxed wakefulness, microawakenings, a rapid eye movement (REM) stage, a first non-REM stage (often referred to as "N1"), a second non-REM stage (often referred to as "N2"), a third non-REM stage (often referred to as "N3"), or any combination thereof. The sleep-wake signal can also be timestamped to indicate a time that the user enters the bed, a time that the user exits the bed, a time that the user attempts to fall asleep, etc. The sleep-wake signal can be measured by the sensor(s) 130 during the sleep session at a predetermined sampling rate, such as, for example, one sample per second, one sample per 30 seconds, one sample per minute, etc. Examples of the one or more sleep-related parameters that can be determined for the user during the sleep session based on the sleep-wake signal include a total time in bed, a total sleep time, a sleep onset latency, a wake-after-sleep-onset parameter, a sleep efficiency, a fragmentation index, or any combination thereof.

Physiological data and/or audio data generated by the one or more sensors 130 can also be used to determine a respiration signal associated with a user during a sleep session. The respiration signal is generally indicative of respiration or breathing of the user during the sleep session. The respiration signal can be indicative of, for example, a respiration rate, a respiration rate variability, an inspiration amplitude, an expiration amplitude, an inspiration-expiration ratio, a number of events per hour, a pattern of events, pressure settings of the respiratory device 122, or any combination thereof. The event(s) can include snoring, apneas, central apneas, obstructive apneas, mixed apneas, hypopneas, a mask leak (e.g., from the user interface 124), a restless leg, a sleeping disorder, choking, an increased heart rate, labored breathing, pain, an asthma attack, an epileptic episode, a seizure, or any combination thereof.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory system 120 and/or ambient pressure. In such implementations, the pressure sensor 132 can be coupled to or integrated in the respiratory device 122. The pressure sensor 132 can be, for example, a capacitive sensor, an electromagnetic sensor, a piezoelectric sensor, a strain-gauge sensor, an optical sensor, a potentiometric sensor, or any combination thereof. In one example, the pressure sensor 132 can be used to determine a blood pressure of a user.

The flow rate sensor 134 outputs flow rate data that can be stored in the memory device 114 and/or analyzed by the processor 112 of the control system 110. In some implementations, the flow rate sensor 134 is used to determine an air flow rate from the respiratory device 122, an air flow rate through the conduit 126, an air flow rate through the user interface 124, or any combination thereof. In such implementations, the flow rate sensor 134 can be coupled to or integrated in the respiratory device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof.

The temperature sensor 136 outputs temperature data that can be stored in the memory device 114 and/or analyzed by the processor 112 of the control system 110. In some implementations, the temperature sensor 136 generates temperatures data indicative of a core body temperature of the user 210 (FIG. 2), a skin temperature of the user 210, a temperature of the air flowing from the respiratory device 122 and/or through the conduit 126, a temperature in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The microphone 140 outputs audio data that can be stored in the memory device 114 and/or analyzed by the processor 112 of the control system 110. The audio data generated by the microphone 140 is reproducible as one or more sound(s) during a sleep session (e.g., sounds from the user 210). The audio data form the microphone 140 can also be used to identify (e.g., using the control system 110) an event experienced by the user during the sleep session, as described in further detail herein. The microphone 140 can be coupled to or integrated in the respiratory device 122, the use interface 124, the conduit 126, or the user device 170.

The speaker 142 outputs sound waves that are audible to a user of the system 100 (e.g., the user 210 of FIG. 2). The speaker 142 can be used, for example, as an alarm clock or to play an alert or message to the user 210 (e.g., in response to an event). In some implementations, the speaker 142 can be used to communicate the audio data generated by the microphone 140 to the user. The speaker 142 can be coupled to or integrated in the respiratory device 122, the user interface 124, the conduit 126, or the user device 170.

The microphone 140 and the speaker 142 can be used as separate devices. In some implementations, the microphone 140 and the speaker 142 can be combined into an acoustic sensor 141, as described in, for example, WO 2018/050913, which serves as background of the invention without forming part thereof. In such implementations, the speaker 142 generates or emits sound waves at a predetermined interval and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user 210 or the bed partner 220 (FIG. 2). Based at least in part on the data from the microphone 140 and/or the speaker 142, the control system 110 can determine a location of the user 210 (FIG. 2) and/or one or more of the sleep-related parameters described in herein.

In some implementations, the sensors 130 include (i) a first microphone that is the same as, or similar to, the microphone 140, and is integrated in the acoustic sensor 141 and (ii) a second microphone that is the same as, or similar to, the microphone 140, but is separate and distinct from the first microphone that is integrated in the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user 210 (FIG. 2) and/or one or more of the sleep-related parameters described herein. An RF receiver (either the RF receiver 146 and the RF transmitter 148 or another RF pair) can also be used for wireless communication between the control system 110, the respiratory device 122, the one or more sensors 130, the user device 170, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147. In some such implementations, the RF sensor 147 includes a control circuit. The specific format of the RF communication can be WiFi, Bluetooth, or the like.

In some implementations, the RF sensor 147 is a part of a mesh system. One example of a mesh system is a WiFi mesh system, which can include mesh nodes, mesh router(s), and mesh gateway(s), each of which can be mobile/movable or fixed. In such implementations, the WiFi mesh system includes a WiFi router and/or a WiFi controller and one or more satellites (e.g., access points), each of which include an RF sensor that the is the same as, or similar to, the RF sensor 147. The WiFi router and satellites continuously communicate with one another using WiFi signals. The WiFi mesh system can be used to generate motion data based on changes in the WiFi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein. For example, the image data from the camera 150 can be used to identify a location of the user, to determine a time when the user 210 enters the bed 230 (FIG. 2), and to determine a time when the user 210 exits the bed 230.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during a sleep session, including a temperature of the user 210 and/or movement of the user 210. The IR sensor 152 can also be used in conjunction with the camera 150 when measuring the presence, location, and/or movement of the user 210. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user 210 (FIG. 2) that can be used to determine one or more sleep-related parameters, such as, for example, a heart rate, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user 210, embedded in clothing and/or fabric that is worn by the user 210, embedded in and/or coupled to the user interface 124 and/or its associated headgear (e.g., straps, etc.), etc.

The ECG sensor 156 outputs physiological data associated with electrical activity of the heart of the user 210. In some implementations, the ECG sensor 156 includes one or more electrodes that are positioned on or around a portion of the user 210 during the sleep session. The physiological data from the ECG sensor 156 can be used, for example, to determine one or more of the sleep-related parameters described herein.

The EEG sensor 158 outputs physiological data associated with electrical activity of the brain of the user 210. In some implementations, the EEG sensor 158 includes one or more electrodes that are positioned on or around the scalp of the user 210 during the sleep session. The physiological data from the EEG sensor 158 can be used, for example, to determine a sleep state of the user 210 at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user 210. The data output by the analyte sensor 174 can be stored in the memory device 114 and used by the control system 110 to determine the identity and concentration of any analytes in the breath of the user 210. In some implementations, the analyte sensor 174 is positioned near a mouth of the user 210 to detect analytes in breath exhaled from the user 210's mouth. For example, when the user interface 124 is a facial mask that covers the nose and mouth of the user 210, the analyte sensor 174 can be positioned within the facial mask to monitor the user 210's mouth breathing. In other implementations, such as when the user interface 124 is a nasal mask or a nasal pillow mask, the analyte sensor 174 can be positioned near the nose of the user 210 to detect analytes in breath exhaled through the user's nose. In still other implementations, the analyte sensor 174 can be positioned near the user 210's mouth when the user interface 124 is a nasal mask or a nasal pillow mask. In this implementation, the analyte sensor 174 can be used to detect whether any air is inadvertently leaking from the user 210's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned near the mouth of the user 210 or within the facial mask (in implementations where the user interface 124 is a facial mask) detects the presence of an analyte, the control system 110 can use this data as an indication that the user 210 is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user 210's face, near the connection between the conduit 126 and the user interface 124, near the connection between the conduit 126 and the respiratory device 122, etc.). Thus, in some implementations, the moisture sensor 176 can be coupled to or integrated in the user interface 124 or in the conduit 126 to monitor the humidity of the pressurized air from the respiratory device 122. In other implementations, the moisture sensor 176 is placed near any area where moisture levels need to be monitored. The moisture sensor 176 can also be used to monitor the humidity of the ambient environment surrounding the user 210, for example, the air inside the bedroom.

The Light Detection and Ranging (LiDAR) sensor 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 166 can measure and map an area extending 5 meters or more away from the sensor. The LiDAR data can be fused with point cloud data estimated by an electromagnetic RADAR sensor, for example. The LiDAR sensor(s) 178 can also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radiotranslucent materials), the LiDAR may reflect off such surfaces, thus allowing a classification of different type of obstacles.

While shown separately in FIG. 1, any combination of the one or more sensors 130 can be integrated in and/or coupled to any one or more of the components of the system 100, including the respiratory device 122, the user interface 124, the conduit 126, the humidification tank 129, the control system 110, the user device 170, or any combination thereof. For example, the microphone 140 and speaker 142 is integrated in and/or coupled to the user device 170 and the pressure sensor 130 and/or flow rate sensor 132 are integrated in and/or coupled to the respiratory device 122. In some implementations, at least one of the one or more sensors 130 is not coupled to the respiratory device 122, the control system 110, or the user device 170, and is positioned generally adjacent to the user 210 during the sleep session (e.g., positioned on or in contact with a portion of the user 210, worn by the user 210, coupled to or positioned on the nightstand, coupled to the mattress, coupled to the ceiling, etc.).

Referring to FIG. 1, the pillow 180 includes a pump device 182 and a multi-compartment bladder 184. The multi-compartment bladder 184 includes a plurality of separate and distinct compartments that can be at least partially inflated and/or at least partially deflated using fluid (e.g., air, water, gas, etc., or any combination thereof). The compartments of the multi-compartment bladder 184 can be inflated and/or deflated by the pump device 182, which generates a pressure differential sufficient to at least partially inflate and/or deflate one or more of the compartments of the multi-compartment bladder 184. In some implementations, each of the compartments in the multi-compartment bladder 184 is directly, fluidly coupled or connected to the pump device 182 (e.g., using a hose, tube, or conduit). In other implementations, all or some of the compartments in the multi-compartment bladder 184 are fluidly coupled or connected together in series (e.g., such that fluid from the pump device 182 enters a first compartment and passes into a second compartment through the first compartment rather than a separate connection between the pump device 182 and the second compartment). Generally, inflation and/or deflation of the compartments of the multi-compartment bladder 184 cause the shape of the pillow 180 to change. In turn, the change in shape of the pillow 180 can cause corresponding movement of a portion of a user that is positioned on and/or in contact with the pillow 180, as described in further detail herein.

The mobile device 170 includes a processor 172, a memory 174, and a display 176. The mobile device 170 can be, for example, a smart phone, a tablet, a laptop, or the like. The processor 172 is the same as, or similar to, the processor 112 of the control system 110. Likewise, the memory 174 is the same as, or similar to, the memory device 114 of the control system 110. The display 176 is generally used to display image(s) including still images, video images, or both. In some implementations, the display 176 acts as a human-machine interface (HMI) that includes a graphic user interface (GUI) configured to display the image(s) and an input interface. The display 176 can be an LED display, an OLED display, an LCD display, or the like. The input interface can be, for example, a touchscreen or touch-sensitive substrate, a mouse, a keyboard, or any sensor system configured to sense inputs made by a human user interacting with the mobile device 170.

While the control system 110 is described and shown in FIG. 1 as being a separate and distinct component of the system 100, in some implementations, the control system 110 is integrated in the mobile device 170 and/or the respiratory system 120. For example, in some implementations, the processor 172 and the memory 174 of the mobile device 170 can be used to perform any of the respective functions described herein for the processor 112 and/or the memory 114 of the control system 110.

While system 100 is shown as including all of the components described above, more or fewer components can be included in a system for adjusting a position of a user during a sleep session. For example, a first alternative system includes the control system 110, the one or more sensors 130, and the pillow 180. As another example, a second alternative system includes the control system 110, the one or more sensors 130, the pillow 180, and the mobile device 170. As yet another example, a third alternative system includes the control system 110, the respiratory system 120, the one or more sensors 130, and the pillow 180. Thus, various systems for adjusting the position of a user during a sleep session can be formed using any portion or portions of the components shown and described herein.

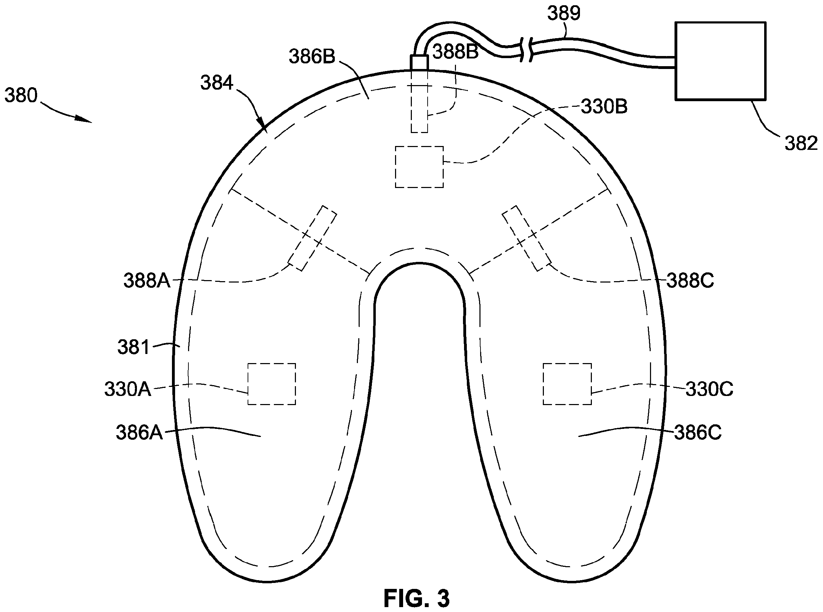

Referring to FIG. 3, a generally U-shaped pillow 380 that is the same as, or similar to, the pillow 180 (FIG. 1) includes an outer cushion 381, a pump device 382, and a multi-compartment bladder 384. The pump device 382 is the same as, or similar to, the pump device 182 of the pillow 180 (FIG. 1) described above. The outer cushion 381 generally surrounds at least a portion the multi-compartment bladder 384 such that a user of the pillow 380 (e.g., the skin of the user) does not come in direct contact with the multi-compartment bladder 384. The outer cushion 381 can include, for example, a fabric material, a foam or memory-foam material, down, feathers, a gel material, or any combination thereof.

The multi-compartment bladder 384 includes a plurality of separate and distinct compartments 386A-386C. The multi-compartment bladder 384 is fluidly coupled or connected to the pump device 382 using a conduit 389 (e.g., tube). The conduit 389 is used to allow fluid (e.g., air) to flow between the multi-compartment bladder 384 and the pump device 382 to cause inflation and/or deflation of the multi-compartment bladder 384. The conduit 389 is coupled to a first valve 388B, which controls the flow of fluid in and/or out of the multi-compartment bladder 384. In some implementations, the first valve 388B is a bi-directional valve that automatically controls fluid flow in either direction (e.g., flow into and out of the multi-compartment bladder 384). For example, the first valve 388B can open or close in response to a predetermined pressure differential. Alternatively, in some implementations, opening and/or closing of the first valve 388B can be pneumatically controlled using, for example, the control system 110, which can be communicatively coupled to the first valve 388B (e.g., using a wired connection or a wireless connection). Further, while the first valve 388B as shown as being in direct fluid communication with the second compartment 386B, more generally, the first valve 388B that receives fluid from conduit 389 can be in direct communication with any of the compartments 386A-386C.

The plurality of compartments 386A-386C are fluidly coupled or connected to one another in series via a second valve 388A and a third valve 388C that are the same as, or similar to, the first valve 388B. The second valve 388A is positioned between the first compartment 386A and the second compartment 386B and thus controls flow between the second compartment 386B and the first compartment 386A. For example, if the first valve 388B is open and the second valve 388B is closed, the second compartment 386B can be inflated or deflated via the conduit 389, whereas the first compartment 386A will not be inflated or deflated. Conversely, if both the first valve 388B and the second valve 388A are open, fluid can flow from the second compartment 386B and into the first compartment 386A to cause inflation or deflation. The third valve 388C is positioned between the second compartment 386B and the third compartment 388C and operates in the same, or similar, manner as the second valve 388A. Each of the valves 388A-388C can be actuated (e.g., opened or closed) in a predetermined sequence to achieve a desired inflation level for each compartment 386A-386C. For example, to inflate the first compartment 386A more than the second compartment 386B, fluid is first pumped into the second compartment 386B via the conduit 389, which thereafter enters the first compartment 386A via the second valve 388A until a predetermined inflation level is reached, and thereafter the second valve 388A can be closed and the second compartment 386B is deflated via the first valve 388B. In this manner, each of the separate and distinct compartments 386A-386C can be inflated by the same or different amounts by controlling the flow of fluid using valves 388A-388C (e.g., the second compartment 386B can be inflated more than the first compartment 386A and/or the third compartment 386C, or vice versa). In some implementations, the valves 388A-388C are passive and do not need to be activated.

The pillow 380 also includes a plurality of sensors 330A-330C that are the same as, or similar to, the one or more sensors 130 (FIG. 1) described above. As shown, a first sensor 330A is coupled to or disposed within the first compartment 386A, a second sensor 330B is coupled to or disposed within the second compartment 386B, and a third sensor 330C is coupled to or disposed within the third compartment 386C. In some implementations, each of the sensors 330A-330C are pressure sensors configured to output pressure data indicative of a pressure within each of the compartments 386A-386B, pressure data indicative of an external pressure applied to an outer portion of the pillow 380 (e.g., by a head of a user), or both. Data from the sensors 330A-330C can be used by the control system 110 to determine whether to inflate or deflate one or more of the compartments 386A-386C (e.g., by comparing the pressure in each compartment 386A-386C to a predetermined threshold pressure). For example, data from the sensors 330A-330C can be used to determine an inflation percentage of each compartment 386A-386C compared to a maximum volume of each compartment 386A-386C (e.g., 10% inflated, 50% inflated, 75% inflated, 100% inflated, etc.) While each compartment 386A-386C is shown as including one of the sensors 330A-330C in FIG. 3, each compartment 386A-386C can include no sensors or a plurality of sensors, including a plurality of different types of sensors (e.g., a pressure sensor and a temperature sensor).

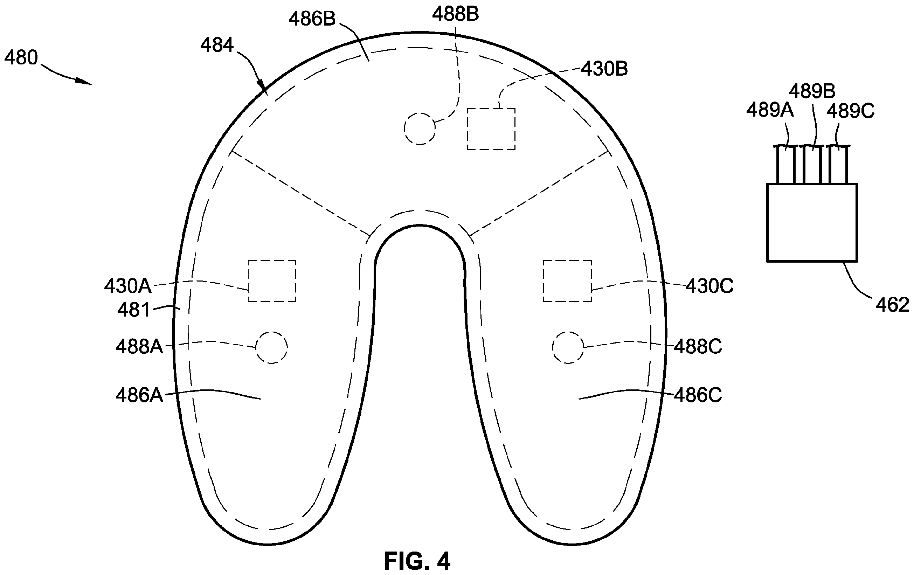

Referring to FIG. 4, a generally U-shaped pillow 480 that is similar to the pillow 380 (FIG. 3) includes an outer cushion 481, a pump device 482, a multi-compartment bladder 484, and a plurality of sensors 430A-430C. The pillow 480 differs from the pillow 380 in that the compartments 486A-486C of the multi-compartment bladder 484 are not fluidly connected in series. Instead, each of the compartments 486A-486C is in direct fluid communication with the pump device 482 via a plurality of conduits 489A-489C and a plurality of valves 488A-488C. Thus, inflation or deflation of each of the compartments 486A-486C can be controlled independently by opening or closing the corresponding valves 488A-488C to directly pump fluid into each compartment 486A-486C from the pump 482.

While the pillow 380 (FIG. 3) and the pillow 480 (FIG. 4) are both shown and described as including three separate and distinct compartments, more generally, the pillow 380 and/or the pillow 480 can include any suitable number of separate and distinct compartments (e.g., 1 compartment, 2 compartments, 5 compartments, 10 compartments, 20 compartments, 50 compartments, 100 compartments, etc.) Moreover, while the pillow 380 (FIG. 3) and the pillow 480 (FIG. 4) are both shown and described as including the same number of valves and the number of compartments, more generally, the pillow 380 (FIG. 3) and/or the pillow 480 (FIG. 4) can include different numbers of valves and compartments (e.g., three compartments and six valves, three compartments and two valves, etc.)

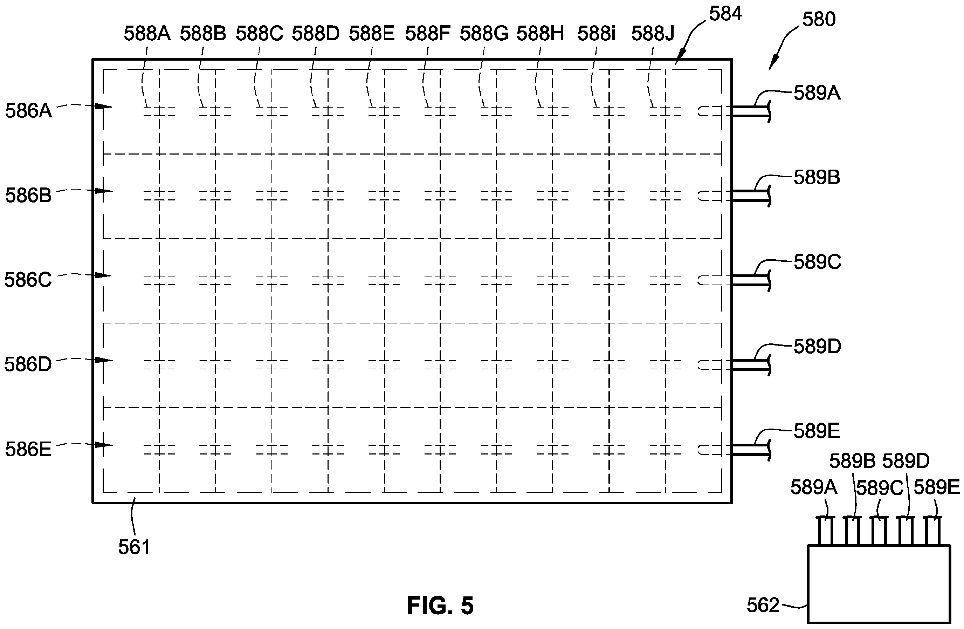

Referring to FIG. 5, a generally rectangular pillow 580 that is the same as, or similar to, the pillow 180 (FIG. 1) includes an outer cushion 581, a pump device 582 and a multi-compartment bladder 584. The pump device 582 is the same as, or similar to, the pump devices of the pillow 180 (FIG. 1), the pillow 380 (FIG. 3), and/or the pillow 480 (FIG. 4). The multi-compartment bladder 584 of the pillow 580 includes a plurality of rows of compartments 586A-586E. Each of the plurality of rows of compartments 586A-586E is fluidly coupled or connected to the pump device 582 via a corresponding one of a plurality of conduits 589A-589E. Further, each of the compartments in each of the plurality of rows 586A-586E are fluidly connected or coupled in series. For example, as shown, the compartments in the first row of compartments 586A are fluidly coupled or connected in series via a plurality of valves 588A-588J. A first one of the rows of compartments 586A is fluidly coupled or connected to the pump device 582 via a first conduit 589A. Actuating the valves 588A-588J (e.g., pneumatically via the control system 110 (FIG. 1)) permits each of the plurality of compartments to be inflated or deflated to a predetermined inflation level. In some implementations, the valves 588A-588J are passive and do not need to be activated.

While the compartments of the multi-compartment bladder 584 of the pillow 580 are shown as being arranged in a matrix including five rows of compartments where each row includes ten compartments, more generally, the multi-compartment bladder 584 can have any ratio of compartments per row to the total number of rows (e.g., 2:1, 2:3, 1:1, 5:1, 10:1, etc.) Even more generally, the multi-compartment bladder 584 can have any number of compartments (e.g., 10 compartments, 50 compartments, 100 compartments, 500 compartments, etc.) Further, while each of the compartments in the multi-compartment bladder 584 are shown as having generally the same size and/or shape, other types and combinations of shapes (e.g., circular shapes, triangular shapes, polygonal shapes, etc. or any combination thereof) and/or relative sizes are expressly contemplated.

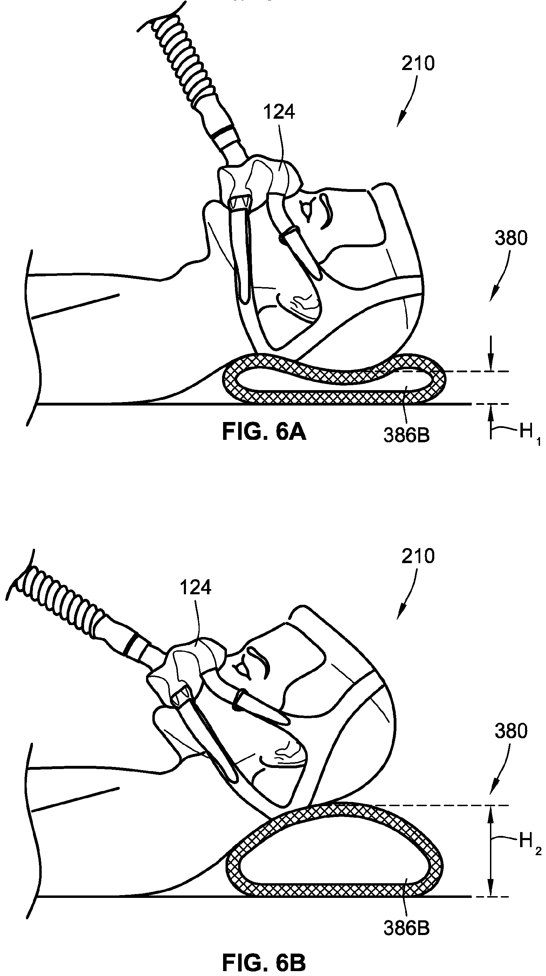

As described herein, the pillow 180 (FIG. 1), the pillow 380 (FIG. 3), the pillow 480 (FIG. 4), and the pillow 580 (FIG. 5) can be used to adjust a position of a user (e.g., a head of the user) during a sleep session by selectively inflating and/or deflating one or more of the compartments of the multi-compartment bladder. For example, referring to FIG. 6A, the user 210 is shown lying on the pillow 380 (FIG. 3) in a first position while wearing the interface 124 of the respiratory system 120 described above. As shown, the second compartment 386B of the multi-compartment bladder 384 has a first height H₁ that is based on the volume of fluid (e.g., air) within the second compartment 386B and the external pressure applied by the head of the user 210. Referring to FIG. 6B, pumping fluid (e.g., air) into the second compartment 386B (via the pump device 382 and conduit 389) causes the second compartment 386B to inflate or expand (countering pressure from the head of the user 210) such that the second compartment 386B has a second height H₂ that is greater than the first height H₁ (FIG. 6A) (e.g., at least 1.5 times greater than the first height, at least 2 times greater than the first height, etc.) As shown by a comparison between FIG. 6A and FIG. 6B, inflating the second compartment 386B causes the user 210 to move from the first position (FIG. 6A) to the second position (FIG. 6B). In the second position, the head of the user 210 is tilted upwards and forwards relative to the first position, which as described herein, can aid in clearing an airway and reducing or eliminating an event (e.g., a mask leak) during a sleep session.

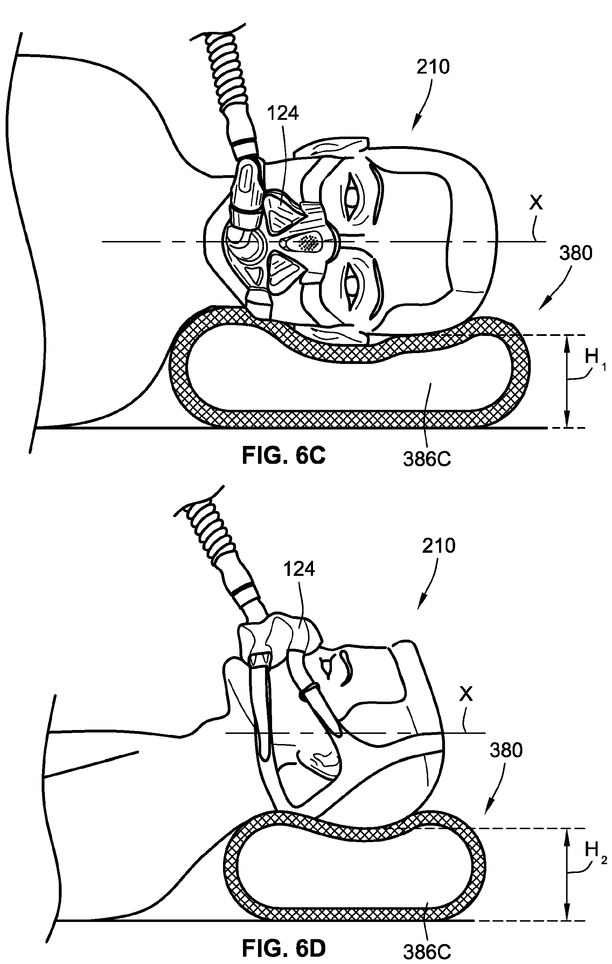

Similarly, referring to FIG. 6C, the user 210 is shown laying on the pillow 380 (FIG. 3) in a third position while wearing the interface 124 of the respiratory system 120 (FIG. 1). In this third position, the user 210 is laying on their side, which as described herein, can cause a gap in the interface between the face of the user 210 and the interface 124, thereby causing air to leak from the interface 124 (e.g., causing noise(s) that disturb sleep, skin/eye irritation, a reduction in the air being delivered into the throat, or any combination thereof). In this position, the third compartment 386C has a first height H₁ that is based on the volume of fluid (e.g., air) within the third compartment 386C and the external pressure applied by the head of the user 210. Referring to FIG. 6D, pumping fluid (e.g., air) into the third compartment 386C (via the pump device 382 and conduit 389) causes the third compartment 386C to inflate or expand (countering pressure from the head of the user 210) such that the third compartment 386C has a second height H₂ that is greater than the first height H₁ (FIG. 6C) (e.g., at least 1.5 times greater than the first height, at least 2 times greater than the first height, etc.) As shown by a comparison between FIG. 6C and FIG. 6D, inflating the third compartment 386C causes the user 210 to move from the third position (FIG. 6A) to a fourth position (FIG. 6B). The head of the user 210 rotates in a circular fashion about a longitudinal axis X when moving between the third position and the fourth position (e.g., the head moves side-to-side). Moving the user 210 from the third position to the fourth position causes the user 210 to be positioned on their back, which can improve the interface (e.g., seal) between the interface 124 and the face of the user 210, thereby reducing or preventing air leakage from the interface 124.

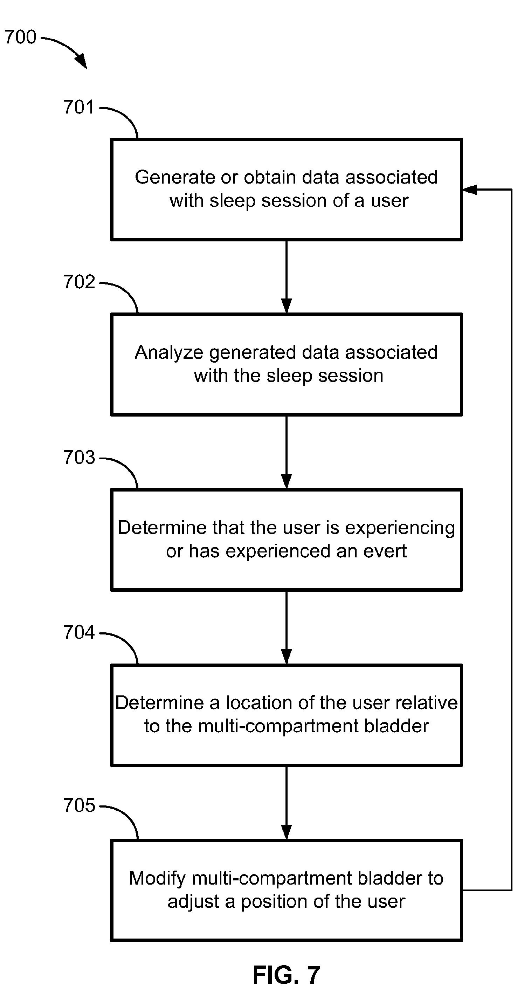

Referring to FIG. 7, a method 700 for adjusting a position of a user during a sleep session is illustrated. One or more of the steps of the method 700 can be implemented using the system 100 (FIG. 1) described herein.

Step 701 of the method 700 includes generating or obtaining data associated with a sleep session of a user. For example, step 701 can include generating or obtaining data using the one or more sensors 130 of the system 100 (FIG. 1) during a sleep session of the user 210 (FIG. 2). The data generated or obtained during step 701 using the one or more sensors 130 (FIG. 1) can include, for example, respiration data indicative of respiration of the user (e.g., a respiration pattern) during the sleep session, temperature data indicative of (i) a temperature of the user 210 (e.g., core temperature and/or skin temperature) during the sleep session, (ii) a temperature of the pillow 180 during the sleep session, (iii) an ambient temperature during the sleep session, or (iv) any combination thereof, movement data indicative of movement of the user 210 during the sleep session, location data indicative of a location of the user 210 (e.g., a relative location of the head of the user 210) during the sleep session, or any combination thereof.

Step 702 of the method 700 includes analyzing the generated data associated with the sleep session that was obtained or generated during step 701 using, for example, the processor 112 of the control system 110 of the system 100 (FIG. 1). For example, step 702 can include analyzing the generated data from step 701 to determine that the user is sleeping (including, for example, a sleep state of the user) or that the user is awake.

Step 703 of the method 700 includes determining, based on the data obtained or generated during step 701 and/or the analysis during step 702, that the user is experiencing or has experienced an event. The event can be, for example, snoring, an apnea such as a positional obstructive sleep apnea, a hypopnea, a restless leg, a sleeping disorder, choking, an increased heart rate, labored breathing, pain such as back pain, an asthma attack, an epileptic episode, a seizure, a mask leak, or any combination thereof. If the user is experiencing or has experienced an event, the method 700 proceeds to step 704. Otherwise, steps 701-703 can be repeated one or more times until an event is detected during 703.

Step 704 of the method 700 includes determining a location of the user 210 (FIG. 2) relative to the multi-compartment bladder 184 (FIG. 1) of the pillow 180 in response to determining that the user is experience or has experienced an event during step 703. For example, step 704 can include determining a location of the head of the user 210 relative to the multi-compartment bladder 184 using the acoustic sensor 141, the camera 150, the infrared sensor 152, or the RF sensor 147 described herein, or any combination thereof. In some implementations, step 704 includes determining an orientation of at least a portion the user 210 relative to the multi-compartment bladder 184. For example, using the location data, the control system 110 can determine that the user 210 is laying on their side (e.g., as shown in FIG. 6C) as opposed to laying on their back (e.g., as shown in FIG. 6D). As another example, using the location data, the control system 110 can determine an angle of the head of the user 210 relative to a horizontal plane (e.g., the mattress and/or the pillow).

Step 705 of the method 700 includes modifying the multi-compartment bladder 184 of the pillow 180 to adjust a position of the user 210. As described herein, modifying (e.g., inflating and/or deflating) one or more of the compartments of the multi-compartment bladder 184 can cause corresponding movement of the user 210. Step 705 includes at least partially inflating at least one of the compartments of the multi-compartment bladder 184, at least partially deflating at least one of the compartments of the multi-compartment bladder 184, or both, to aid in adjusting a position of the user 210.

As described herein, in some implementations, the system 100 does not include the respiratory system 120 or the user is not wearing the interface 124. In such implementations, step 705 can include modifying the multi-compartment bladder 184 to adjust the position of the user responsive to identifying a positional event (e.g., positional apnea, positional snoring, etc.). Step 705 can also include modifying the multi-compartment bladder 184 to adjust the position of the user responsive to determining that the current position of the user is associated with a positional event (e.g., based on previously recorded data for the user) to aid in preventing or reducing the likelihood of the user experiencing the positional event.

In some implementations, the system 100 includes the respiratory system 120, and the user is wearing the interface 124, and step 705 can include modifying the multi-compartment bladder 184 to adjust the position of the user responsive to identifying a positional event (e.g., positional apnea, positional snoring, etc.) and substantially maintaining and/or reducing the pressure of the air delivered to the user 210 in response to detection of the positional event. Subsequent to the multi-compartment bladder being modified in step 705, steps 701-703 can be repeated to determine if the user 210 is still experiencing the positional event, in which case the pressure of the air delivered to the user 210 can be increased to treat the positional event. Furthermore, adjusting the position of the user 210 can advantageously allow use of increased air pressure by, for example, moving the head and/or body of the user 210 to a position more conducive to better mask seal (see, for example, the above discussion in relation to FIGS. 6C and 6D).

In some implementations, step 705 includes modifying the multi-compartment bladder 184 according to a predetermined inflation scheme that is associated with the determined event. For example, if the determined event is snoring or choking, which can be caused by a narrowed or closed airway, the multi-compartment bladder 184 can be modified according to a fill scheme that tilts the head of the user 210 forward and/or upwards to aid in clearing and/or opening the airway (e.g., inflating the second compartment 386B of the pillow 380 to cause the user 210 to move from a first position (FIG. 6A) to a second position (FIG. 6B) as described herein).

In some implementations, subsequent to the multi-compartment bladder being modified in step 705, steps 701-703 are repeated to determine if the user 210 is still experiencing the event. If the user is still experiencing the event, step 705 can be repeated one or more times to continue to modify the multi-compartment bladder as described above until the user is no longer experiencing the event. In such implementations, the inflation scheme coinciding with termination of the ongoing event can be stored in the memory device 114 (FIG. 1) and associated with that event. As such, in the future, the multi-compartment bladder 184 can be inflated according to that fill scheme in step 705 in response to determining that the user 210 is or has experienced the associated event. In other words, the initial inflation scheme can be adjusted based on continued analysis of the data associated with the sleep session.

In some implementations, the method 700 further includes displaying a sleep score indicative of the quality of the sleep session (e.g., on the display 176 of the mobile device 170). In such implementations, the sleep score can be the same as, or similar to, the ones described in International Publication No. WO 2015/006364 A2 (e.g., paragraphs [0278]-[0285]), which serves as background of the invention without forming part thereof. The sleep score can be determined (e.g., by the control system 110) based on the data from steps 701-703. Generally, the sleep score can be expressed as a number (e.g., an integer) between 0 and 100, where a score of 100 is indicative of very high quality sleep, a score of 0 is indicative of very poor quality sleep, and scores of 1-99 represent varying qualities therebetween. The sleep score can be based on, for example, a number or type of events during the sleep session, the duration of the sleep session, the duration of one or more sleep states during the sleep session (e.g., the relative durations of REM sleep and/or non-REM sleep) the amount of movement of the user during the sleep session, or any combination thereof. Advantageously, the sleep score permits the user to compare the quality of their sleep over the course of one or more sleep sessions and make adjustments to improve the quality of sleep and the associated sleep score. The sleep score can also be displayed in a manner that illustrates how the use of the multi-compartment bladder has impacted the sleep score of the user over time (e.g., the sleep score is improved during sleep sessions when the user sleeps with the multi-compartment bladder compared to sleep sessions where the user does not sleep with the multi-compartment bladder.

In some implementations, the respiratory system 120 (FIG. 1) is not used during the method 700 (e.g., the user 210 is not wearing the interface 124). In other implementations, the method 700 can be performed when the user 210 is using the respiratory system 120. As described herein, the respiratory system 120 can be used to address one or more events that occur during a sleep session. For example, responsive to determining that the user is experiencing an event, one or more settings of the respiratory system 120 can be adjusted (e.g., the predetermined pressure from the respirator device 122 can be increased). In implementations of the method 700 where the respiratory system 120 is used, adjustment of the respiratory system 120 settings can occur if the modification of the multi-compartment bladder in step 705 does not adequately address (e.g., terminate) the determined event. In other words, in some implementations, the control system 110 responds to a determined event by first adjusting the position of the user 210 using the multi-compartment bladder 184 (step 705), and if the event does not terminate, the control system 110 responds by adjusting the settings of the respiratory system 120 (e.g., adjusting the air pressure, adjusting the flow rate, adjusting the temperature). For example, the control system 110 can optimally adjust the settings of the respiratory system 120 to match the physiological data (e.g., breathing/respiration pattern) generated or obtained prior to the event. Thus, advantageously, the respiratory system 120 settings are only adjusted (e.g., by increasing the air pressure) if repositioning the user 210 via the multi-compartment bladder 184 being activated fails to address the determined event.

Alternatively, in some implementations of the method 700, step 705 can occur simultaneously with, or subsequent to, the adjustment of one or more settings of the respiratory system 120 (e.g., increasing the pressure) responsive to determining that the user 210 is experiencing or has experienced an event (step 703).

In some implementations, events can be monitored (such as snoring events monitored by an acoustic sensor) relative to the position of the user 210 and/or the inflation status of the multi-compartment bladder 184. Such monitoring can be to learn, for example, via a machine learning model, the user position(s) and/or the inflation status, which results in a reduced number of events, a reduced number of arousals, optimized sleep patterns, or any combination thereof. Similarly, in some implementations, the actuation and inflation status of the multi-compartment bladder 184 can be monitored, along with the user's sleep state (such as wakefulness, relaxed wakefulness, micro-awakenings, a rapid eye movement (REM) stage, a first non-REM stage (often referred to as "N1"), a second non-REM stage (often referred to as "N2"), a third non-REM stage (often referred to as "N3"), determined by, for example, a sleep-wake signal, as described herein), to learn how the actuation and inflation status of the multi-compartment bladder 184 affects the user's sleep in terms of sleep cycles, wake times, etc., and thereby how to minimize the effects on the user's sleep caused by the multi-compartment bladder 184. For example, if actuation of the multi-compartment bladder 184 early in a sleep session, and/or in a particular sleep state, results in awakenings, then actuation of the multi-compartment bladder 184 during those times can be minimized and/or avoided for that user.

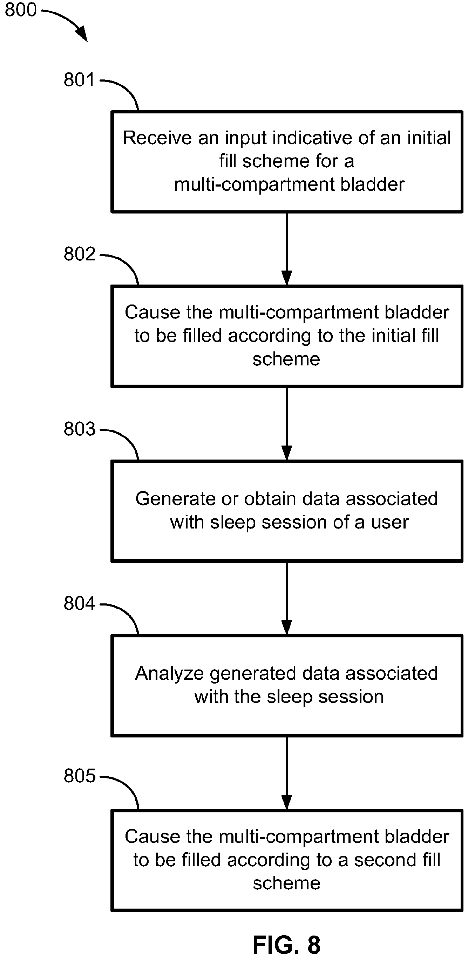

Referring to FIG. 8, a method 800 for adjusting a multi-compartment bladder from an initial fill scheme to a second fill scheme is illustrated. The method 800 can be implemented using the system 100 (FIG. 1) described herein.

Step 801 of the method 800 includes receiving an input indicative of an initial fill scheme for the multi-compartment bladder 184. The input can be received by the control system 110 from the mobile device 170, which in turn receives the input from the user 210. The initial fill scheme of the multi-compartment bladder 184 can be, for example, a fill scheme that is comfortable for the user to assist the user in falling asleep. Alternatively, the initial fill scheme can be a fill scheme that positions the user to watch TV or to read while lying in their bed. In some implementations, the initial fill scheme can be customized by the user 210 using the mobile device 170 (e.g., the user 210 can select or adjust an inflation level for one or more of the compartments of the multi-compartment bladder 184) and thereafter stored in the memory device 114 of the control system 110 and/or the memory 174 of the mobile device 170.

Step 802 of the method 800 includes causing the multi-compartment bladder 184 to be filled according to the initial fill scheme (step 801). To cause the multi-compartment bladder 184 to assume the initial fill scheme, step 802 includes at least partially inflating at least one of the compartments of the multi-compartment bladder 184, at least partially deflating at least one of the compartments of the multi-compartment bladder 184, or both, to achieve the initial fill scheme.

Step 803 of the method 800 is the same as, or similar to, step 701 of method 700 (FIG. 7) and includes generating or obtaining data associated with a sleep session of a user. Step 804 of the method 800 is the same as, or similar to, step 702 of method 700 (FIG. 7) and includes analyzing the generated or obtained data from step 803 that is associated with the sleep session of the user.

Step 805 of the method 800 includes causing the multi-compartment bladder 184 to be filled according to a second fill scheme that is different than the initial fill scheme (step 802). For example, while the initial fill scheme can be selected by the user 210 for comfort to aid in falling asleep, the initial fill scheme may not optimally position the user 210 during a sleep session (e.g., in a position that aids in preventing or reducing events, such as a supine position which can aid in avoiding certain mask leaks). In some implementations, the second fill scheme can be associated with one or more events and/or one or more sleep states. For example, responsive to determining that the user is experiencing or has experienced an event, step 805 includes causing the multi-compartment bladder 184 to be filled according to a second fill scheme that is associated with the determined event. Similarly, responsive to determining that the user is in one of a plurality of sleep states, step 805 includes causing the multi-compartment bladder 184 to be filled according to a second fill scheme that is associated with the determined sleep state. Alternatively, step 805 can be performed automatically at a predetermined time subsequent to determining that the user 210 is asleep.

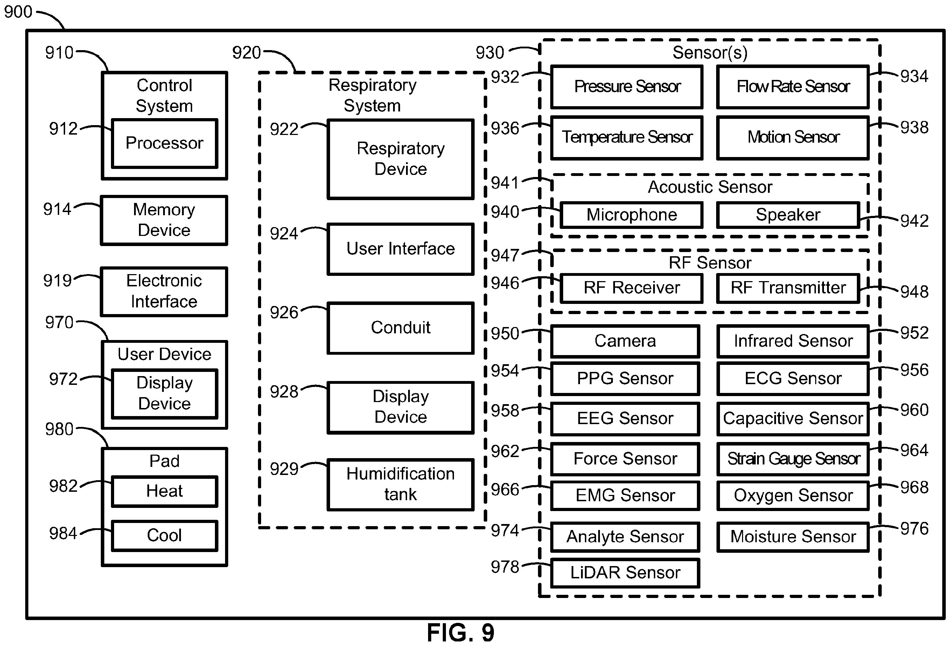

Referring to FIG. 9, a system 900 that is similar to the system 100 (FIG. 1) described above includes a control system 910, a respiratory system 920, one or more sensors 930, and a mobile device 970. The control system 910 includes a processor 912 and a memory device 914 that are the same as, or similar to, the processor 112 and the memory device 114 (FIG. 1) described above. The respiratory system 920 includes a respirator device 922, a mask 924, a humidification tank 926, and a tube 928 that are the same as, or similar to, the respirator device 122, the mask 924, the humidification tank 126, and the tube 128 of the respiratory system 120 (FIG. 1) described above. The one or more sensors 930 are the same as, or similar to, the one or more sensors 130 (FIG. 1). The mobile device 970 includes a processor 972, a memory device 974, and a display 976 that are the same as, or similar to, the processor 172, the memory device 174, and the display 176 of the mobile device 170 (FIG. 1) described above.

The system 900 mainly differs from the system 100 (FIG. 1) in that the system 900 includes a pad 960 including a heating element 962 and a cooling element 964 instead of a pillow (e.g., pillow 180). The pad 960 can also include a cushion made of a fabric material, a foam or memory-foam material, a gel material, or any combination thereof. In some implementations, the system 900 can be modified to have the pad 960 coupled to, integrated in, or placed on any one of the pillow 180 (FIG. 1), the pillow 380 (FIG. 3), the pillow 480 (FIG. 4), or the pillow 560 (FIG. 5) described herein, such that a pillow is also included in the system 900. In some such implementations, the pad 960 can be a pillow.

The heating element 962 is generally used to heat the pad 960 to a predetermined temperature or within a range of predetermined temperatures. The heating element 962 can be, for example, an insulated resistance wire or coil, carbon fiber, a ceramic or semiconductor, or any combination thereof. The cooling element 964 is generally used to heat the pad 960 to a predetermined temperature or within a range of predetermined temperatures. The cooling element 964 can be, for example, one or more fans, a thermoelectric cooling device, a Peliter cooler, a heat exchanger, or any combination thereof. The heating element 962 and/or the cooling element 964 can be communicatively coupled to and control by (e.g., actuated by) the control system 910. The pad 960 can also include a temperature sensor that is the same as, or similar to, the temperature sensor 934 for determining the temperature of the pad 960.

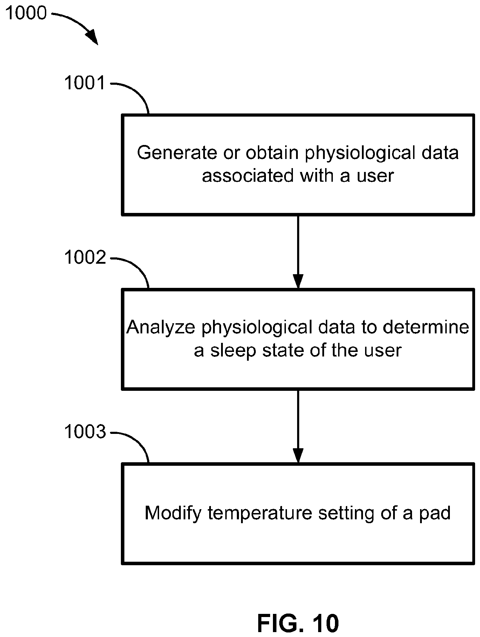

Referring to FIG. 10, a method 1000 for modifying a temperature of the pad 960 to induce or prolong one or more sleep states is illustrated. The method 1000 can be implemented using the system 900 (FIG. 9) described above.

Step 1001 of the method 1000 includes generating or obtaining physiological data associated with a user using the one or more sensors 930 (FIG. 9). Examples of the physiological data can include respiration rate, breath analysis, airway resistance, blood flow to brain, blood pressure, brain activity, heart rate, sympathetic nerve activity, blood alcohol level, activity (e.g., body movement, chest movement, limb movement, body position, coughing in bed), blood oxygen saturation (SpO2), expired carbon dioxide (CO₂), or any combination thereof. The physiological data can include temperature-related information, such as, for example, a skin temperature of the user, a core body temperature of the user, an ambient temperature, a pillow temperature, or any combination thereof. This physiological data obtained or generated during step 1001 can be received and stored in the memory 914 of the control system 910 for analysis during step 1002.

Step 1002 of the method 1000 includes analyzing the physiological data obtained or generated during step 1001 to determine a sleep state of the user using the control system 910. Examples of different sleep states include wakefulness, relaxed wakefulness, light sleep, deep sleep, or rapid eye movement (REM) (including, for example, phasic REM sleep, tonic REM sleep, deep REM sleep, and/or light REM sleep).

Step 1003 of the method 1000 includes modifying a temperature setting of the pad 960 based on the sleep state of the user determined during step 1002 to induce or prolong one or more of the sleep states described above. Each of these sleep states described above can be associated with temperature data based on previously recorded sleep states or a predetermined relationship. For example, each sleep state can be associated with a range of core body temperatures, a range of skin temperatures, a range of pillow temperatures, a range of ambient temperatures, or any combination thereof. Step 1003 can include lowering the temperature of the pad 960 using the cooling element 964 responsive to determining that the skin temperature, the core body temperature, the pillow temperature, or any combination thereof exceeds a predetermined temperature threshold or range of values associated with a desired sleep state. Conversely, step 1003 can include raising the temperature of the pad 960 using the heating element 962 responsive to determining that the skin temperature or core body temperature is lower than a predetermined threshold or range of values associated with a desired sleep state.

Steps 1001-1003 of the method 1000 can be repeated one or more times throughout a sleep session (e.g., over a 3-hour period, a 5-hour period, an 8-hour period, etc.) to dynamically change the temperature of the pad 960 based on a desired sleep state at any given time within the sleep session. Alternatively steps 1001-1003 can be repeated one or more times through a sleep session to maintain a predetermined temperature of the pad 960 despite changing ambient temperature conditions.

While the multi-compartment bladders have been described herein as being used to adjust a position of a user responsive to determining that the user is experiencing or has experienced a sleep-related event, the same or similar multi-compartment bladders can be used to aid in preventing or reducing the likelihood of infant flat-head syndrome by adjusting a position of the infant (e.g., the head of the infant). The multi-compartment bladders described herein can also be used to adjust a position of a user that is confined to a bed to prevent and/or reduce the likelihood of the user developing bed sores, back pain, etc., or any combination thereof. Furthermore, the multi-compartment bladders described herein can be used to balance the need to treat and/or prevent events, such as apnea events (e.g., positional events), mask leaks, etc., with the need to reduce and/or avoid development of bed sores, back pain, etc. For example, when no events are detected or predicted, the multi-compartment bladder can periodically adjust the position of a user to reduce or avoid development of bed sores, back pain, etc. In other examples, when no bed sores, back pain, etc. are detected or reported, the multi-compartment bladder can adjust the position of a user to treat or prevent events at a frequency and duration as required.

As used herein, a sleep session can be defined in a number of ways based on, for example, an initial start time and an end time. Referring to FIG. 3, an exemplary timeline 300 for a sleep session is illustrated. The timeline 300 includes an enter bed time (t_{bed}), a go-to-sleep time (t_{GTS}), an initial sleep time (tₛₗₑₑₚ), a first micro-awakening MA₁ and a second micro-awakening MA₂, a wake-up time (t_{wake}), and a rising time (tᵣᵢₛₑ).

As used herein, a sleep session can be defined in multiple ways. For example, a sleep session can be defined by an initial start time and an end time. In some implementations, a sleep session is a duration where the user is asleep, that is, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

In some implementations, a sleep session is defined as the entire time between the time in the evening at which the user first entered the bed, and the time the next morning when user last left the bed. Put another way, a sleep session can be defined as a period of time that begins on a first date (e.g., Monday, January 6, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, January 7, 2020) at a second time (e.g., 7:00 AM), that can be referred to as the next morning, when the user first exits the bed with the intention of not going back to sleep that next morning.

In some implementations, the user can manually define the beginning of a sleep session and/or manually terminate a sleep session. For example, the user can select (e.g., by clicking or tapping) one or more user-selectable element that is displayed on the display device 172 of the user device 170 (FIG. 1) to manually initiate or terminate the sleep session.

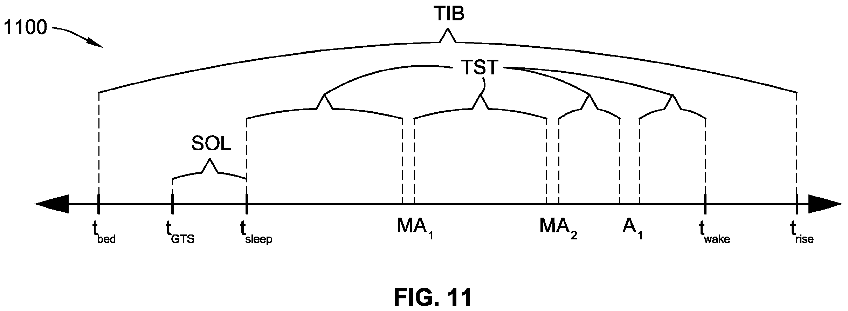

Referring to FIG. 11, an exemplary timeline 1100 for a sleep session is illustrated. The timeline 1100 includes an enter bed time (t_{bed}), a go-to-sleep time (t_{GTS}), an initial sleep time (tₛₗₑₑₚ), a first micro-awakening MA₁, a second micro-awakening MA₂, an awakening A, a wake-up time (t_{wake}), and a rising time (tᵣᵢₛₑ).

The enter bed time t_{bed} is associated with the time that the user initially enters the bed (e.g., bed 230 in FIG. 2) prior to falling asleep (e.g., when the user lies down or sits in the bed). The enter bed time t_{bed} can be identified based on a bed threshold duration to distinguish between times when the user enters the bed for sleep and when the user enters the bed for other reasons (e.g., to watch TV). For example, the bed threshold duration can be at least about 10 minutes, at least about 20 minutes, at least about 30 minutes, at least about 45 minutes, at least about 1 hour, at least about 2 hours, etc. While the enter bed time t_{bed} is described herein in reference to a bed, more generally, the enter time t_{bed} can refer to the time the user initially enters any location for sleeping (e.g., a couch, a chair, a sleeping bag, etc.).

The go-to-sleep time (GTS) is associated with the time that the user initially attempts to fall asleep after entering the bed (t_{bed}). For example, after entering the bed, the user may engage in one or more activities to wind down prior to trying to sleep (e.g., reading, watching TV, listening to music, using the user device 170, etc.). The initial sleep time (tₛₗₑₑₚ) is the time that the user initially falls asleep. For example, the initial sleep time (tₛₗₑₑₚ) can be the time that the user initially enters the first non-REM sleep stage.

The wake-up time t_{wake} is the time associated with the time when the user wakes up without going back to sleep (e.g., as opposed to the user waking up in the middle of the night and going back to sleep). The user may experience one of more unconscious microawakenings (e.g., microawakenings MA₁ and MA₂) having a short duration (e.g., 5 seconds, 10 seconds, 30 seconds, 1 minute, etc.) after initially falling asleep. In contrast to the wake-up time t_{wake}, the user goes back to sleep after each of the microawakenings MA₁ and MA₂. Similarly, the user may have one or more conscious awakenings (e.g., awakening A) after initially falling asleep (e.g., getting up to go to the bathroom, attending to children or pets, sleep walking, etc.). However, the user goes back to sleep after the awakening A. Thus, the wake-up time t_{wake} can be defined, for example, based on a wake threshold duration (e.g., the user is awake for at least 15 minutes, at least 20 minutes, at least 30 minutes, at least 1 hour, etc.).

Similarly, the rising time tᵣᵢₛₑ is associated with the time when the user exits the bed and stays out of the bed with the intent to end the sleep session (e.g., as opposed to the user getting up during the night to go to the bathroom, to attend to children or pets, sleep walking, etc.). In other words, the rising time tᵣᵢₛₑ is the time when the user last leaves the bed without returning to the bed until a next sleep session (e.g., the following evening). Thus, the rising time tᵣᵢₛₑ can be defined, for example, based on a rise threshold duration (e.g., the user has left the bed for at least 15 minutes, at least 20 minutes, at least 30 minutes, at least 1 hour, etc.). The enter bed time t_{bed} time for a second, subsequent sleep session can also be defined based on a rise threshold duration (e.g., the user has left the bed for at least 4 hours, at least 6 hours, at least 8 hours, at least 12 hours, etc.).

As described above, the user may wake up and get out of bed one more times during the night between the initial t_{bed} and the final tᵣᵢₛₑ. In some implementations, the final wake-up time t_{wake} and/or the final rising time tᵣᵢₛₑ that are identified or determined based on a predetermined threshold duration of time subsequent to an event (e.g., falling asleep or leaving the bed). Such a threshold duration can be customized for the user. For a standard user which goes to bed in the evening, then wakes up and goes out of bed in the morning any period (between the user waking up (t_{wake}) or raising up (tᵣᵢₛₑ), and the user either going to bed (t_{bed}), going to sleep (T_{GTS}) or falling asleep (tₛₗₑₑₚ) of between about 12 and about 18 hours can be used. For users that spend longer periods of time in bed, shorter threshold periods may be used (e.g., between about 8 hours and about 14 hours). The threshold period may be initially selected and/or later adjusted based on the system monitoring the user's sleep behavior.

The total time in bed (TIB) is the duration of time between the time enter bed time t_{bed} and the rising time tᵣᵢₛₑ. The total sleep time (TST) is associated with the duration between the initial sleep time and the wake-up time, excluding any conscious or unconscious awakenings and/or micro-awakenings therebetween. Generally, the total sleep time (TST) will be shorter than the total time in bed (TIB) (e.g., one minute short, ten minutes shorter, one hour shorter, etc.). For example, referring to the timeline 1100 of FIG. 11, the total sleep time (TST) spans between the initial sleep time tₛₗₑₑₚ and the wake-up time t_{wake}, but excludes the duration of the first micro-awakening MA₁, the second micro-awakening MA₂, and the awakening A. As shown, in this example, the total sleep time (TST) is shorter than the total time in bed (TIB).

In some implementations, the total sleep time (TST) can be defined as a persistent total sleep time (PTST). In such implementations, the persistent total sleep time excludes a predetermined initial portion or period of the first non-REM stage (e.g., light sleep stage). For example, the predetermined initial portion can be between about 30 seconds and about 20 minutes, between about 1 minute and about 10 minutes, between about 3 minutes and about 5 minutes, etc. The persistent total sleep time is a measure of sustained sleep, and smooths the sleep-wake hypnogram. For example, when the user is initially falling asleep, the user may be in the first non-REM stage for a very short time (e.g., about 30 seconds), then back into the wakefulness stage for a short period (e.g., one minute), and then goes back to the first non-REM stage. In this example, the persistent total sleep time excludes the first instance (e.g., about 30 seconds) of the first non-REM stage.

In some implementations, the sleep session is defined as starting at the enter bed time (t_{bed}) and ending at the rising time (tᵣᵢₛₑ), i.e., the sleep session is defined as the total time in bed (TIB). In some implementations, a sleep session is defined as starting at the initial sleep time (tₛₗₑₑₚ) and ending at the wake-up time (t_{wake}). In some implementations, the sleep session is defined as the total sleep time (TST). In some implementations, a sleep session is defined as starting at the go-to-sleep time (t_{GTS}) and ending at the wake-up time (t_{wake}). In some implementations, a sleep session is defined as starting at the go-to-sleep time (T_{GTS}) and ending at the rising time (tᵣᵢₛₑ). In some implementations, a sleep session is defined as starting at the enter bed time (t_{bed}) and ending at the wake-up time (t_{wake}). In some implementations, a sleep session is defined as starting at the initial sleep time (tₛₗₑₑₚ) and ending at the rising time (tᵣᵢₛₑ).

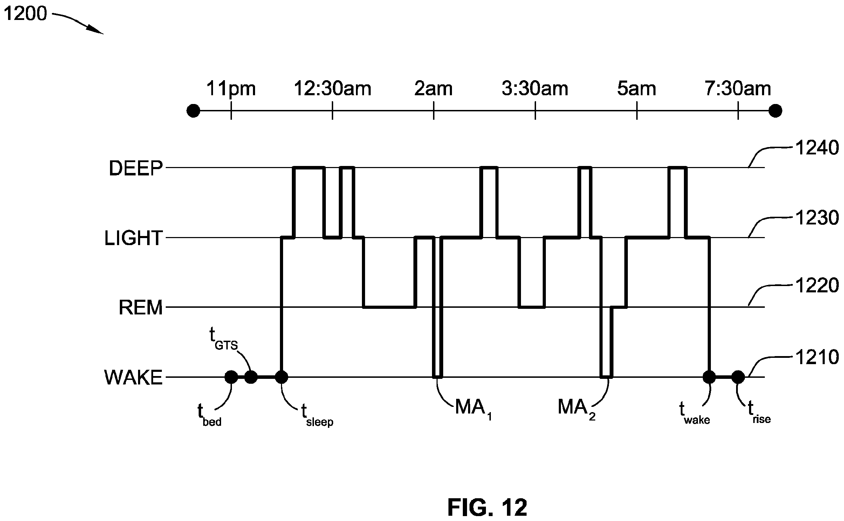

Referring to FIG. 12, an exemplary hypnogram 1200 corresponding to the timeline 300 (FIG. 3), according to some implementations, is illustrated. As shown, the hypnogram 400 includes a sleep-wake signal, a wakefulness stage axis 1210, a REM stage axis 1220, a light sleep stage axis 1230, and a deep sleep stage axis 1240. The intersection between the sleep-wake signal and one of the axes 1210-1240 is indicative of the sleep stage at any given time during the sleep session.

The sleep-wake signal can be generated based on physiological data associated with the user (e.g., generated by one or more of the sensors 130 described herein). The sleep-wake signal can be indicative of one or more sleep states, including wakefulness, relaxed wakefulness, microawakenings, a REM stage, a first non-REM stage, a second non-REM stage, a third non-REM stage, or any combination thereof. In some implementations, one or more of the first non-REM stage, the second non-REM stage, and the third non-REM stage can be grouped together and categorized as a light sleep stage or a deep sleep stage. For example, the light sleep stage can include the first non-REM stage and the deep sleep stage can include the second non-REM stage and the third non-REM stage. While the hypnogram 1200 is shown in FIG. 12 as including the light sleep stage axis 430 and the deep sleep stage axis 1240, in some implementations, the hypnogram 1200 can include an axis for each of the first non-REM stage, the second non-REM stage, and the third non-REM stage. In other implementations, the sleep-wake signal can also be indicative of a respiration signal, a respiration rate, an inspiration amplitude, an expiration amplitude, an inspiration-expiration ratio, a number of events per hour, a pattern of events, or any combination thereof. Information describing the sleep-wake signal can be stored in the memory device 114.

The hypnogram 1200 can be used to determine one or more sleep-related parameters, such as, for example, a sleep onset latency (SOL), wake-after-sleep onset (WASO), a sleep efficiency (SE), a sleep fragmentation index, sleep blocks, or any combination thereof.

The sleep onset latency (SOL) is defined as the time between the go-to-sleep time (T_{GTS}) and the initial sleep time (tₛₗₑₑₚ). In other words, the sleep onset latency is indicative of the time that it took the user to actually fall asleep after initially attempting to fall asleep. In some implementations, the sleep onset latency is defined as a persistent sleep onset latency (PSOL). The persistent sleep onset latency differs from the sleep onset latency in that the persistent sleep onset latency is defined as the duration time between the go-to-sleep time and a predetermined amount of sustained sleep. In some implementations, the predetermined amount of sustained sleep can include, for example, at least 10 minutes of sleep within the second non-REM stage, the third non-REM stage, and/or the REM stage with no more than 2 minutes of wakefulness, the first non-REM stage, and/or movement therebetween. In other words, the persistent sleep onset latency requires up to, for example, 8 minutes of sustained sleep within the second non-REM stage, the third non-REM stage, and/or the REM stage. In other implementations, the predetermined amount of sustained sleep can include at least 10 minutes of sleep within the first non-REM stage, the second non-REM stage, the third non-REM stage, and/or the REM stage subsequent to the initial sleep time. In such implementations, the predetermined amount of sustained sleep can exclude any micro-awakenings (e.g., a ten second micro-awakening does not restart the 10-minute period).

The wake-after-sleep onset (WASO) is associated with the total duration of time that the user is awake between the initial sleep time and the wake-up time. Thus, the wake-after-sleep onset includes short and micro-awakenings during the sleep session (e.g., the microawakenings MA₁ and MA₂ shown in FIG. 12) whether conscious or unconscious. In some implementations, the wake-after-sleep onset (WASO) is defined as a persistent wake-after-sleep onset (PWASO) that only includes the total durations of awakenings having a predetermined length (e.g., greater than 10 seconds, greater than 30 seconds, greater than 60 seconds, greater than about 5 minutes, greater than about 10 minutes, etc.)

The sleep efficiency (SE) is determined as a ratio of the total time in bed (TIB) and the total sleep time (TST). For example, if the total time in bed is 8 hours and the total sleep time is 7.5 hours, the sleep efficiency for that sleep session is 93.75%. The sleep efficiency is indicative of the sleep hygiene of the user. For example, if the user enters the bed and spends time engaged in other activities (e.g., watching TV) before sleep, the sleep efficiency will be reduced (e.g., the user is penalized). In some implementations, the sleep efficiency (SE) can be calculated based on the total time in bed (TIB) and the total time that the user is attempting to sleep. In such implementations, the total time that the user is attempting to sleep is defined as the duration between the go-to-sleep (GTS) time and the rising time described herein. For example, if the total sleep time is 8 hours (e.g., between 11 PM and 7 AM), the go-to-sleep time is 10:45 PM, and the rising time is 7:15 AM, in such implementations, the sleep efficiency parameter is calculated as about 94%.

The fragmentation index is determined based at least in part on the number of awakenings during the sleep session. For example, if the user had two micro-awakenings (e.g., micro-awakening MA₁ and micro-awakening MA₂ shown in FIG. 12), the fragmentation index can be expressed as 2. In some implementations, the fragmentation index is scaled between a predetermined range of integers (e.g., between 0 and 10).

The sleep blocks are associated with a transition between any stage of sleep (e.g., the first non-REM stage, the second non-REM stage, the third non-REM stage, and/or the REM) and the wakefulness stage. The sleep blocks can be calculated at a resolution of, for example, 30 seconds.

In some implementations, the systems and methods described herein can include generating or analyzing a hypnogram including a sleep-wake signal to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof based at least in part on the sleep-wake signal of a hypnogram.

In other implementations, one or more of the sensors 130 can be used to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof, which in turn define the sleep session. For example, the enter bed time t_{bed} can be determined based on, for example, data generated by the motion sensor 138, the microphone 140, the camera 150, or any combination thereof. The go-to-sleep time can be determined based on, for example, data from the motion sensor 138 (e.g., data indicative of no movement by the user), data from the camera 150 (e.g., data indicative of no movement by the user and/or that the user has turned off the lights), data from the microphone 140 (e.g., data indicative of the using turning off a TV), data from the user device 170 (e.g., data indicative of the user no longer using the user device 170), data from the pressure sensor 132 and/or the flow rate sensor 134 (e.g., data indicative of the user turning on the respiratory device 122, data indicative of the user donning the user interface 124, etc.), or any combination thereof.

## Claims

1. A system (100) comprising:
one or more sensors (130) configured to generate data associated with a sleep session of a user (210);
a multi-compartment bladder (184) configured to be positioned adjacent to the user during the sleep session;
a memory (114) storing machine-readable instructions; and
a control system (110) including one or more processors (112) configured to execute the machine-readable instructions to:
determine, based at least in part on the generated data, that the user is sleeping;
responsive to the determination that the user is sleeping, activate the multi-compartment bladder;
determine, based at least in part on the generated data, that the user is experiencing or has experienced an event; and
responsive to the determination that the user is experiencing or has experienced the event, cause the multi-compartment bladder to be modified to aid in causing the user to move.

2. The system of claim 1, wherein the multi-compartment bladder has a generally U-shape and includes at least three separate and distinct compartments that are (i) fluidly coupled in series or (ii) configured to be separately inflated.

3. The system of claim 2, wherein the multi-compartment bladder is caused to be modified such that a head of the user, when positioned within the generally U-shaped multi-compartment bladder, is moved in a generally circular fashion.

4. The system of any one of claims 1 to 3, wherein the one or more sensors include a pressure sensor configured to measure a pressure within at least one of the compartments of the multi-compartment bladder and the control system is further configured to execute the machine-readable instructions to determine, based at least in part on data from the pressure sensor, a location of a head of the user relative to the multi-compartment bladder.

5. The system of any one of claims 1 to 4, wherein the one or more sensors include a location-based sensor coupled to each of the compartments of the multi-compartment bladder and the control system is further configured to execute the machine-readable instructions to determine, based at least in part on an output of the location-based sensors, a location of a head of the user relative to the multi-compartment bladder.

6. The system of any one of claims 1 to 5, wherein the one or more sensor includes a microphone (140), a speaker (142), an RF transmitter (148), an RF receiver (146), or any combination thereof.

7. The system of any one of claims 1 to 6, further comprising a pump (182) coupled to the multi-compartment bladder, wherein the multi-compartment bladder has at least two separate and distinct compartments, and the modifying the multi-compartment bladder includes (i) at least partially inflating at least one of the compartments of the multi-compartment bladder, (ii) at least partially deflating at least one of the compartments of the multi-compartment bladder, or (iii) both (i) and (ii).

8. The system of claim 7, wherein the control system is further configured to determine a location of a head of the user relative to the multi-compartment bladder during the sleep session based at least in part on the generated data and the modifying the multi-compartment bladder is based at least in part on the determined location of the head of the user relative to the multi-compartment bladder.

9. The system of any one of claims 1 to 8, wherein the modifying includes causing the multi-compartment bladder to be modified according to a first inflation scheme and the control system is further configured to execute the machine-readable instructions to, (i) subsequent to the multi-compartment bladder being modified according to the first inflation scheme, continue to analyze the generated data to determine if the user is still experiencing the event and (ii) responsive to the continued analysis of the generated data resulting in a determination that the user is still experiencing the event, cause the multi-compartment bladder to be modified according to a second inflation scheme.

10. The system of claim 9, wherein the control system is further configured to execute the machine-readable instructions to, (i) subsequent to the multi-compartment bladder being modified according to the first inflation scheme, continue to analyze the generated data and (ii) based at least in part on the continued analysis, modify one or more aspects of the first inflation scheme.

11. The system of any one of claims 1 to 10, wherein the control system is further configured to:
receive an input indicative of an initial fill scheme for the multi-compartment bladder;
responsive to the received input, cause the multi-compartment bladder to be filled according to the initial fill scheme; and
cause the multi-compartment bladder to be modified such that the multi-compartment bladder is filled according to a second fill scheme.

12. The system of any one of claims 1 to 11, further comprising:
A respiratory device (122) configured to supply pressurized air; and
a user interface (124) coupled to the respirator device via a conduit (126) and being configured to engage the user during the sleep session to aid in directing the supplied pressurized air to an airway of the user.

13. The system of claim 12, wherein the control system is further configured to:
determine, based at least in part on the generated data, whether the user is still experiencing the event subsequent to the multi-compartment bladder being modified; and
cause a pressure setting of the respirator device to be modified responsive to determining that the user is still experiencing the event.

14. The system of any one of claims 1 to 13, wherein the event includes an apnea, a hypopnea, a restless leg, a sleeping disorder, choking, an increased heart rate, labored breathing, an asthma attack, an epileptic episode, a seizure, or any combination thereof and does not include snoring.

15. A method for use in the system according to any of the preceding claims, the method comprising:
receiving, from the one or more sensors (130), data associated with a sleep session of a user;
determining, based at least in part on the data, that the user is sleeping;
responsive to determining that the user is sleeping, activating the multi-compartment bladder (184);
determining, based at least in part on the data, that the user is experiencing or has experienced an event during the sleep session; and
causing the multi-compartment bladder that is positioned adjacent to the user to be modified aid in causing the user to move responsive to determining that the user is experiencing or has experienced the event.

## Patentansprüche

1. System (100), umfassend:
einen oder mehrere Sensoren (130), die dazu ausgelegt sind, Daten zu erzeugen, die mit einer Schlafsitzung eines Benutzers (210) assoziiert sind;
eine Mehrkammerblase (184), die dazu ausgelegt ist, während der Schlafsitzung benachbart zum Benutzer positioniert zu werden;
einen Speicher (114), der maschinenlesbare Anweisungen speichert; und
ein Steuersystem (110), das einen oder mehrere Prozessoren (112) umfasst, die dazu ausgelegt sind, die maschinenlesbaren Anweisungen für Folgendes auszuführen:
Bestimmen zumindest teilweise basierend auf den erzeugten Daten, dass der Benutzer schläft;
Aktivieren der Mehrkammerblase in Reaktion auf die Bestimmung, dass der Benutzer schläft;
Bestimmen zumindest teilweise basierend auf den erzeugten Daten, dass der Benutzer ein Ereignis erlebt oder erlebt hat; und
Veranlassen, dass die Mehrkammerblase modifiziert wird, um dabei zu helfen, den Benutzer zu veranlassen, sich zu bewegen, in Reaktion auf die Bestimmung, dass der Benutzer das Ereignis erlebt oder erlebt hat, zu veranlassen.

2. System nach Anspruch 1, wobei die Mehrkammerblase im Allgemeinen eine U-Form aufweist und mindestens drei getrennte und unterschiedliche Kammern umfasst, die (i) fluidisch in Reihe gekoppelt oder (ii) dazu ausgelegt sind, getrennt aufgeblasen werden.

3. System nach Anspruch 2, wobei veranlasst wird, dass die Mehrkammerblase so modifiziert wird, dass der Kopf des Benutzers, wenn innerhalb der im Allgemeinen U-förmigen Mehrkammerblase positioniert, in einer im Allgemeinen kreisförmigen Weise bewegt wird.

4. System nach einem der Ansprüche 1 bis 3, wobei der eine oder die mehreren Sensoren einen Drucksensor umfassen, der dazu ausgelegt ist, einen Druck innerhalb mindestens einer der Kammern der Mehrkammerblase messen, und das Steuersystem ferner dazu ausgelegt ist, die maschinenlesbaren Anweisungen auszuführen, um zumindest teilweise basierend auf Daten vom Drucksensor eine Lage des Kopfes des Benutzers relativ zu der Mehrkammerblase zu bestimmen.

5. System nach einem der Ansprüche 1 bis 4, wobei der eine oder die mehreren Sensoren einen lagebasierten Sensor umfassen, der mit jeder der Kammern der Mehrkammerblase gekoppelt ist, und das Steuersystem ferner dazu ausgelegt ist, die maschinenlesbaren Anweisungen auszuführen, um zumindest teilweise basierend auf einer Ausgabe der lagebasierten Sensoren die Lage des Kopfs des Benutzers in Bezug auf die Mehrkammerblase zu bestimmen.

6. System nach einem der Ansprüche 1 bis 5, wobei der eine oder die mehreren Sensoren ein Mikrofon (140), einen Lautsprecher (142), einen HF-Sender (148), einen HF-Empfänger (146) oder eine beliebige Kombination davon umfassen.

7. System nach einem der Ansprüche 1 bis 6, ferner umfassend eine Pumpe (182), die mit der Mehrkammerblase gekoppelt ist, wobei die Mehrkammerblase mindestens zwei getrennte und unterschiedliche Kammern aufweist und das Modifizieren der Mehrkammerblase (i) zumindest teilweises Aufblasen mindestens einer der Kammern der Mehrkammerblase, (ii) zumindest teilweises Ablassen von Luft aus mindestens einer der Kammern der Mehrkammerblase oder (iii) sowohl (i) als auch (ii) umfasst.

8. System nach Anspruch 7, wobei das Steuersystem ferner dazu ausgelegt ist, eine Lage des Kopfs des Benutzers relativ zu der Mehrkammerblase während der Schlafsitzung zumindest teilweise basierend auf den erzeugten Daten zu bestimmen, und das Modifizieren der Mehrkammerblase zumindest teilweise auf der bestimmten Lage des Kopfs des Benutzers relativ zu der Mehrkammerblase basiert.

9. System nach einem der Ansprüche 1 bis 8, wobei das Modifizieren ein Veranlassen umfasst, dass die Mehrkammerblase gemäß einem ersten Aufblasschema modifiziert wird, und das Steuersystem ferner dazu ausgelegt ist, die maschinenlesbaren Anweisungen auszuführen, um (i) nach dem Modifizieren der Mehrkammerblase gemäß dem ersten Aufblasschema die Analyse der erzeugten Daten fortzusetzen, um zu bestimmen, ob der Benutzerdas Ereignis immer noch erlebt, und (ii) in Reaktion auf die fortgesetzte Analyse der erzeugten Daten, die zu einer Bestimmung führt, dass der Benutzer das Ereignis immer noch erlebt, die Modifizierung der Mehrkammerblase gemäß einem zweiten Aufblasschema zu veranlassen.

10. System nach Anspruch 9, wobei das Steuersystem ferner dazu ausgelegt ist, die maschinenlesbaren Anweisungen auszuführen, um (i) nach dem Modifizieren der Mehrkammerblase gemäß dem ersten Aufblasschema die Analyse der erzeugten Daten fortzusetzen, und (ii) zumindest teilweise basierend auf der fortgesetzten Analyse einen oder mehrere Aspekte des ersten Aufblasschemas zu modifizieren.

11. System nach einem der Ansprüche 1 bis 10, wobei das Steuersystem ferner ausgelegt ist zum:
Empfangen einer Eingabe, die eine anfängliches Füllschema für die Mehrkammerblase angibt;
Veranlassen in Reaktion auf die empfangene Eingabe, dass die Mehrkammerblase gemäß dem anfänglichen Füllschema gefüllt wird; und
Veranlassen, dass die Mehrkammerblase so modifiziert wird, dass die Mehrkammerblase gemäß einem zweiten Füllschema gefüllt wird.

12. System nach einem der Ansprüche 1 bis 11, ferner umfassend:
eine Atemvorrichtung (122), die zum Zuführen von Druckluft ausgelegt ist; und
eine Benutzerschnittstelle (124), die über eine Leitung (126) mit der Atemvorrichtung gekoppelt und dazu ausgelegt ist, während der Schlafsitzung mit dem Benutzer verbunden zu werden, um dabei zu helfen, die zugeführte Druckluft in die Luftröhre des Benutzers zu leiten.

13. System nach Anspruch 12, wobei das Steuersystem ferner ausgelegt ist zum:
Bestimmen zumindest teilweise basierend auf den erzeugten Daten, ob der Benutzer das Ereignis nach dem Modifizieren der Mehrkammerblase immer noch erlebt; und
Veranlassen, dass eine Druckeinstellung der Atemvorrichtung modifiziert wird, in Reaktion auf ein Bestimmen, dass der Benutzer das Ereignis immer noch erlebt.

14. System nach einem der Ansprüche 1 bis 13, wobei das Ereignis eine Apnoe, eine Hypopnoe, ein Schnittstellenleck, ein unruhiges Bein, eine Schlafstörung, Ersticken, eine erhöhte Herzfrequenz, erschwertes Atmen, einen Asthmaanfall, einen epileptischen Anfall, einen Krampfanfall oder eine beliebige Kombination davon umfasst und Schnarchen nicht umfasst.

15. Verfahren zur Herstellung eines Systems nach einem der vorhergehenden Ansprüche, wobei das Verfahren Folgendes umfasst:
Empfangen von Daten, die mit einer Schlafsitzung eines Benutzers assoziiert sind, von dem einen oder den mehreren Sensoren (130);
Bestimmen zumindest teilweise basierend auf den erzeugten Daten, dass der Benutzer schläft;
Aktivieren der Mehrkammerblase (184) in Reaktion auf das Bestimmen, dass der Benutzer schläft;
Bestimmen zumindest teilweise basierend auf den erzeugten Daten, dass der Benutzer ein Ereignis erlebt oder erlebt hat; und
Veranlassen, dass die benachbart zum Benutzer positionierte Mehrkammerblase modifiziert wird, um dabei zu helfen, den Benutzer zu veranlassen, sich zu bewegen, in Reaktion auf das Bestimmen, das der Benutzer das Ereignis erlebt oder erlebt hat.

## Revendications

1. Système (100) comprenant :
un ou plusieurs capteurs (130) configurés pour générer des données associées à une session de sommeil d'un utilisateur (210) ;
une vessie à compartiments multiples (184) configurée pour être positionnée de manière adjacente à l'utilisateur pendant la session de sommeil ;
une mémoire (114) stockant des instructions lisibles par machine ; et
un système de commande (110) incluant un ou plusieurs processeurs (112) configurés pour exécuter les instructions lisibles par machine pour :
déterminer, sur la base au moins en partie des données générées, que l'utilisateur dort ;
en réponse à la détermination du fait que l'utilisateur dort, activer la vessie à compartiments multiples ;
déterminer, sur la base au moins en partie des données générées, que l'utilisateur vit ou a vécu un événement ; et
en réponse à la détermination du fait que l'utilisateur vit ou a vécu l'événement, amener la vessie à compartiments multiples à être modifiée pour aider à faire bouger l'utilisateur.

2. Système selon la revendication 1, dans lequel la vessie à compartiments multiples présente une forme globalement en U et inclut au moins trois compartiments séparés et distincts qui sont (i) couplés fluidiquement en série ou (ii) configurés pour être gonflés séparément.

3. Système selon la revendication 2, dans lequel la vessie à compartiments multiples est amenée à être modifiée de sorte qu'une tête de l'utilisateur, lorsqu'elle est positionnée à l'intérieur de la vessie à compartiments multiples globalement en forme de U, soit déplacée de manière globalement circulaire.

4. Système selon l'une quelconque des revendications 1 à 3, dans lequel le ou les plusieurs capteurs incluent un capteur de pression configuré pour mesurer une pression à l'intérieur d'au moins l'un des compartiments de la vessie à compartiments multiples et le système de commande est en outre configuré pour exécuter les instructions lisibles par machine pour déterminer, sur la base au moins en partie de données provenant du capteur de pression, un emplacement d'une tête de l'utilisateur par rapport à la vessie à compartiments multiples.

5. Système selon l'une quelconque des revendications 1 à 4, dans lequel le ou les plusieurs capteurs incluent un capteur basé sur la localisation couplé à chacun des compartiments de la vessie à compartiments multiples et le système de commande est en outre configuré pour exécuter les instructions lisibles par machine pour déterminer, sur la base au moins en partie d'une sortie des capteurs basés sur la localisation, un emplacement d'une tête de l'utilisateur par rapport à la vessie à compartiments multiples.

6. Système selon l'une quelconque des revendications 1 à 5, dans lequel le ou les plusieurs capteurs incluent un microphone (140), un haut-parleur (142), un émetteur RF (148), un récepteur RF (146), ou toute combinaison de ceux-ci.

7. Système selon l'une quelconque des revendications 1 à 6, comprenant en outre une pompe (182) couplée à la vessie à compartiments multiples, dans lequel la vessie à compartiments multiples présente au moins deux compartiments séparés et distincts, et la modification de la vessie à compartiments multiples inclut (i) le gonflage au moins partiel d'au moins l'un des compartiments de la vessie à compartiments multiples, (ii) le dégonflage au moins partiel d'au moins l'un des compartiments de la vessie à compartiments multiples, ou (iii) à la fois (i) et (ii).

8. Système selon la revendication 7, dans lequel le système de commande est en outre configuré pour déterminer un emplacement d'une tête de l'utilisateur par rapport à la vessie à compartiments multiples pendant la session de sommeil sur la base au moins en partie des données générées et la modification de la vessie à compartiments multiples est basée au moins en partie sur l'emplacement déterminé de la tête de l'utilisateur par rapport à la vessie à compartiments multiples.

9. Système selon l'une quelconque des revendications 1 à 8, dans lequel la modification inclut le fait d'amener la vessie à compartiments multiples à être modifiée selon un premier schéma de gonflage et le système de commande est en outre configuré pour exécuter les instructions lisibles par machine pour, (i) après modification de la vessie à compartiments multiples selon le premier schéma de gonflage, continuer à analyser les données générées pour déterminer si l'utilisateur vit toujours l'événement et (ii) en réponse à l'analyse continue des données générées aboutissant à une détermination selon laquelle l'utilisateur vit toujours l'événement, amener la vessie à compartiments multiples à être modifiée selon un second schéma de gonflage.

10. Système selon la revendication 9, dans lequel le système de commande est en outre configuré pour exécuter les instructions lisibles par machine pour, (i) après modification de la vessie à compartiments multiples selon le premier schéma de gonflage, continuer à analyser les données générées et (ii) sur la base au moins en partie de l'analyse continue, modifier un ou plusieurs aspects du premier schéma de gonflage.

11. Système selon l'une quelconque des revendications 1 à 10, dans lequel le système de commande est en outre configuré pour :
recevoir une entrée indicative d'un schéma de remplissage initial pour la vessie à compartiments multiples ;
en réponse à l'entrée reçue, amener la vessie à compartiments multiples à être remplie selon le schéma de remplissage initial ; et
amener la vessie à compartiments multiples à être modifiée de sorte que la vessie à compartiments multiples soit remplie selon un second schéma de remplissage.

12. Système selon l'une quelconque des revendications 1 à 11, comprenant en outre :
un dispositif respiratoire (122) configuré pour fournir de l'air sous pression ; et
une interface utilisateur (124) couplée au dispositif respiratoire via un conduit (126) et configurée pour engager l'utilisateur pendant la session de sommeil pour aider à diriger l'air sous pression fourni vers une voie respiratoire de l'utilisateur.

13. Système selon la revendication 12, dans lequel le système de commande est en outre configuré pour :
déterminer, sur la base au moins en partie des données générées, si l'utilisateur vit toujours l'événement après la modification de la vessie à compartiments multiples ; et
amener un réglage de pression du dispositif respiratoire à être modifié en réponse à la détermination du fait que l'utilisateur vit toujours l'événement.

14. Système selon l'une quelconque des revendications 1 à 13, dans lequel l'événement inclut une apnée, une hypopnée, un syndrome des jambes sans repos, un trouble du sommeil, une suffocation, une augmentation de la fréquence cardiaque, une respiration laborieuse, une crise d'asthme, un épisode épileptique, une crise, ou toute combinaison de ceux-ci et n'inclut pas le ronflement.

15. Procédé destiné à être utilisé dans le système selon l'une quelconque des revendications précédentes, le procédé comprenant :
la réception, à partir du ou des plusieurs capteurs (130), de données associées à une session de sommeil d'un utilisateur ;
la détermination, sur la base au moins en partie des données, du fait que l'utilisateur dort ;
en réponse à la détermination du fait que l'utilisateur dort, l'activation de la vessie à compartiments multiples (184) ;
la détermination, sur la base au moins en partie des données, du fait que l'utilisateur vit ou a vécu un événement pendant la session de sommeil ; et
le fait d'amener la vessie à compartiments multiples qui est positionnée de manière adjacente à l'utilisateur à être modifiée, aidant à faire bouger l'utilisateur en réponse à la détermination du fait que l'utilisateur vit ou a vécu l'événement.
